# EUROPEAN PATENT SPECIFICATION

(11) **EP 4 181 811 B1**
(45) Date of publication and mention of the grant of the patent: **25.02.2026**
(21) Application number: 21752266.3
(22) Date of filing: 20.07.2021
(51) Int. Cl.: A61B 34/10, A61B 34/20, A61B 34/00, A61B 34/30, A61B 90/00, A61B 34/32

(54) **MEDICAL ARM CONTROL SYSTEM**
MEDIZINISCHES ARMSTEUERUNGSSYSTEM
SYSTÈME DE COMMANDE DE BRAS MÉDICAL

(30) Priority: 20.07.2020 JP 2020124036
(43) Date of publication of application: 24.05.2023
(73) Proprietor: Sony Group Corporation, Tokyo 108-0075 (JP)
(72) Inventor: USUI, Masaru, Tokyo 108-0075 (JP); KITO, Takashi, Tokyo 108-0075 (JP); MATSUDA, Yasuhiro, Tokyo 108-0075 (JP); WAKANA, Kazuhito, Tokyo 108-0075 (JP); TOMATSU, Kei, Tokyo 108-0075 (JP)
(74) Representative: D Young & Co LLP
(86) International application number: PCT/JP2021/027237
(87) International publication number: WO 2022/019318

(56) References cited:
- CN-A- 109 531 566
- US-A1- 2015 094 736
- US-A1- 2019 090 969

## Description

### Technical Field

This application claims the benefit of Japanese Priority Patent Application JP 2020-124036 filed on July 20, 2020.

The present disclosure relates to a medical arm control system, a medical arm control method, and a program.

### Background

In recent years, endoscopic surgery is performed by imaging an abdominal cavity of a patient by using an endoscope and displaying an image thus taken by the endoscope on a display. For example, Patent Literature 1 below discloses a technique for linking control of an arm that supports an endoscope with control of electronic zoom of the endoscope.

### Citation List

### Patent Literature

PTL 1: International Publication No. 2017/145475

PTL2: CN109531566A describes a medical arm with autonomous operation control.

### Summary of Invention

The invention is defined by the appended independent claim. Further embodiments of the invention are illustrated in the dependent claims. The methods described in the present disclosure do not form part of the invention, but are useful for understanding the invention.

### Summary of Disclosure Technical Problem

In recent years, in a medical observation system, techniques for allowing a robot arm device that supports an endoscope to autonomously operate have been developed. For example, there is an attempt to allow a robot arm device to autonomously control movement (scope work) of an endoscope during surgery so that a surgeon can perform a procedure by using a surgical instrument in the environment.

However, since a state of a body internal environment differs from one patient to another, it is inevitable that an unknown environment exists in actual surgery even if a plurality of cases are learned in advance in order to perform autonomous operation.

Therefore, the present disclosure proposes a medical arm control system, a medical arm control method, and a program capable of generating autonomous operation control information for a robot arm device suitable for various body internal environments in real time.

### Solution to Problem

According to the present disclosure, a medical arm control system is provided. The medical arm control system includes: a control information generation unit that generates autonomous operation control information for causing a medical arm to autonomously operate based on external input information; a simulation unit that performs an operation simulation of the medical arm; and a correction unit that corrects the autonomous operation control information in real time based on a result of the operation simulation of the medical arm.

Further, a medical arm control system may include circuitry configured to generate autonomous operation control information to autonomously operate a medical arm based on external input information, simulate an operation performed using the medical arm, and correct the autonomous operation control information in real time based on a result of the simulation of the operation of the medical arm.

Also, according to the present disclosure, a medical arm control method is provided. The medical arm control method includes causing a medical arm control device to: generate autonomous operation control information for causing a medical arm to autonomously operate based on external input information; perform an operation simulation of the medical arm; and correct the autonomous operation control information in real time based on a result of the operation simulation of the medical arm.

Further, a medical arm control method may include generating autonomous operation control information to autonomously operate a medical arm based on external input information, performing a simulation of an operation using the medical arm, and correcting the autonomous operation control information in real time based on a result of the simulation.

Moreover, according to the present disclosure, a program is provided. The program allows a computer to function as: a control information generation unit that generates autonomous operation control information for causing a medical arm to autonomously operate based on external input information; a simulation unit that performs an operation simulation of the medical arm; and a correction unit that corrects the autonomous operation control information in real time based on a result of the operation simulation of the medical arm.

Additionally, according to the present disclosure, a simulator for correcting autonomous operation control information for a medical arm may include circuitry configured to simulate an operation performed using the medical arm, and correct the autonomous operation control information in real time based on a result of the simulation of the operation of the medical arm.

Further additionally, according to the present disclosure, a method for generating a learning model for a medical arm for a reference operation in a reference environment may include generating an autonomous operation learning model using machine learning based on external input information regarding the medical arm in the reference operation as learning data, generating autonomous operation rules by analyzing the external input information used as the learning data, and generating a reference body internal environment map based on the external input information used as the learning data, the autonomous operation learning model, the autonomous operation rules, and the reference body internal environment map serving as the learning model for the medical arm.

### Brief Description of Drawings

[Fig.1] FIG. 1 illustrates an example of a schematic configuration of an endoscopic surgery system to which the technique according to the present disclosure can be applied.
[Fig.2] FIG. 2 is a block diagram illustrating an example of a functional configuration of a camera head and a camera control unit (CCU) illustrated in FIG. 1.
[Fig.3] FIG. 3 is a schematic view illustrating a configuration of an oblique-viewing endoscope according to an embodiment of the present disclosure.
[Fig.4] FIG. 4 is a block diagram illustrating an example of a configuration of a medical observation system according to the embodiment of the present disclosure.
[Fig.5] FIG. 5 is a block diagram illustrating an example of a configuration of a learning model generation unit according to the embodiment of the present disclosure.
[Fig.6] FIG. 6 is a flowchart of a control method in a learning model generation stage according to the embodiment of the present disclosure.
[Fig.7] FIG. 7 is a block diagram illustrating an example of a configuration of an autonomous operation execution unit according to the embodiment of the present disclosure.
[Fig.8] FIG. 8 is a block diagram illustrating an example of a configuration of a presenting device according to the embodiment of the present disclosure.
[Fig.9] FIG. 9 is a flowchart (No. 1) of a control method in an autonomous operation execution stage according to the embodiment of the present disclosure.
[Fig.10A] FIG. 10A is a sub-flowchart of step S200 illustrated in FIG. 9.
[Fig.10B] FIG. 10B futher illustrates parallel steps of FIG. 10A for clarity.
[Fig.10C] FIG. 10C futher illustrates parallel steps of FIG. 10A for clarity.
[Fig.10D] FIG. 10D futher illustrates parallel steps of FIG. 10A for clarity.
[Fig.11] FIG. 11 is an explanatory diagram (No. 1) for explaining details of the control method according to the embodiment of the present disclosure.
[Fig.12] FIG. 12 is an explanatory diagram (No. 2) for explaining the details of the control method according to the embodiment of the present disclosure.
[Fig.13] FIG. 13 is an explanatory diagram (No. 3) for explaining the details of the control method according to the embodiment of the present disclosure.
[Fig.14] FIG. 14 is an explanatory diagram (No. 4) for explaining the details of the control method according to the embodiment of the present disclosure.
[Fig.15] FIG. 15 is an explanatory diagram (No. 5) for explaining the details of the control method according to the embodiment of the present disclosure.
[Fig.16] FIG. 16 is an explanatory diagram (No. 6) for explaining the details of the control method according to the embodiment of the present disclosure.
[Fig.17] FIG. 17 is an explanatory diagram (No. 7) for explaining the details of the control method according to the embodiment of the present disclosure.
[Fig.18] FIG. 18 is an explanatory diagram (No. 8) for explaining the details of the control method according to the embodiment of the present disclosure.
[Fig.19] FIG. 19 is an explanatory diagram (No. 9) for explaining the details of the control method according to the embodiment of the present disclosure.
[Fig.20] FIG. 20 is an explanatory diagram (No. 10) for explaining the details of the control method according to the embodiment of the present disclosure.
[Fig.21] FIG. 21 is a sub-flowchart of step S300 illustrated in FIG. 9.
[Fig.22] FIG. 22 is an explanatory diagram (No. 11) for explaining the details of the control method according to the embodiment of the present disclosure.
[Fig.23] FIG. 23 is an explanatory diagram (No. 12) for explaining the details of the control method according to the embodiment of the present disclosure.
[Fig.24] FIG. 24 is an explanatory diagram (No. 13) for explaining the details of the control method according to the embodiment of the present disclosure.
[Fig.25] FIG. 25 is an explanatory diagram (No. 14) for explaining the details of the control method according to the embodiment of the present disclosure.
[Fig.26] FIG. 26 is a flowchart (No. 2) of a control method in the autonomous operation execution stage according to the embodiment of the present disclosure.
[Fig.27] FIG. 27 is an explanatory diagram (No. 15) for explaining the details of the control method according to the embodiment of the present disclosure.
[Fig.28] FIG. 28 is a hardware configuration diagram illustrating an example of a computer that realizes functions of the autonomous operation execution unit according to the embodiment of the present disclosure.

### Description of Embodiments

Hereinafter, embodiments of the present disclosure will be described in detail with reference to the accompanying drawings. In the present specification and the drawings, constituent elements having substantially identical functional configurations are given identical reference signs, and repeated description thereof is omitted. Further, in the present specification and the drawings, a plurality of constituent elements having substantially identical or similar functional configurations may be distinguished by adding different alphabets after identical reference signs. However, a plurality of constituent elements having substantially identical or similar functional configurations are given only identical reference signs when they need not be distinguished.

Embodiments of the present technology are described below with reference to the drawings. The present disclosure may be embodied as a system, a method, and/or a computer program. The methods and systems described herein may be implemented using computer programming or engineering techniques including computer software, firmware, hardware or any combination or subset thereof.

The computer program may include a computer readable storage medium on which computer readable program instructions are recorded that may cause one or more processors to carry out aspects of the embodiment. The computer readable storage medium may be a tangible device that can store instructions for use by an instruction execution device (processor). The computer readable storage medium may be, for example, but is not limited to, an electronic storage device, a magnetic storage device, an optical storage device, an electromagnetic storage device, a semiconductor storage device, or any appropriate combination of these devices.

Computer readable program instructions described in this disclosure can be downloaded to an appropriate computing or processing device from a computer readable storage medium or to an external computer or external storage device via a global network (i.e., the Internet), a local area network, a wide area network and/or a wireless network.

Aspects of the present disclosure are described herein with reference to flow diagrams and block diagrams of methods, apparatus (systems), and computer programs according to embodiments of the disclosure. It will be understood by those skilled in the art that each block of the flow diagrams and block diagrams, and combinations of blocks in the flow diagrams and block diagrams, can be implemented by computer readable program instructions.

The computer readable program instructions may also be loaded onto a computer, other programmable apparatus, or other device to cause a series of operational steps to be performed on the computer, other programmable apparatuses, or other devices to produce a computer implemented process, such that the instructions which execute on the computer, other programmable apparatus, or other device implement the functions specified in the flow diagrams and block diagrams in the present disclosure.

As used herein, "unit" refers to circuitry that may be configured via the execution of computer readable instructions, and the circuitry may include one or more local processors (e.g., CPU's), and/or one or more remote processors, such as a cloud computing resource, or any combination thereof.

The description will be given in the following order.
1. Example of configuration of endoscopic surgery system 5000
1.1 Schematic configuration of endoscopic surgery system 5000
1.2 Example of detailed configuration of support arm device 5027
1.3 Example of detailed configuration of light source device 5043
1.4 Example of detailed configuration of camera head 5005 and CCU 5039
1.5 Example of configuration of endoscope 5001
2. Medical observation system
3. Background leading to embodiment of present disclosure
4. Embodiment
4.1 Example of detailed configuration of learning model generation unit 100
4.2 Control method in learning model generation stage
4.3 Example of detailed configuration of autonomous operation execution unit 200
4.4 Control method in autonomous operation execution stage
4.5 Modifications
5. Hardware configuration
6. Supplement

### <<1. Example of configuration of endoscopic surgery system 5000>>

### <1.1 Schematic configuration of endoscopic surgery system 5000>

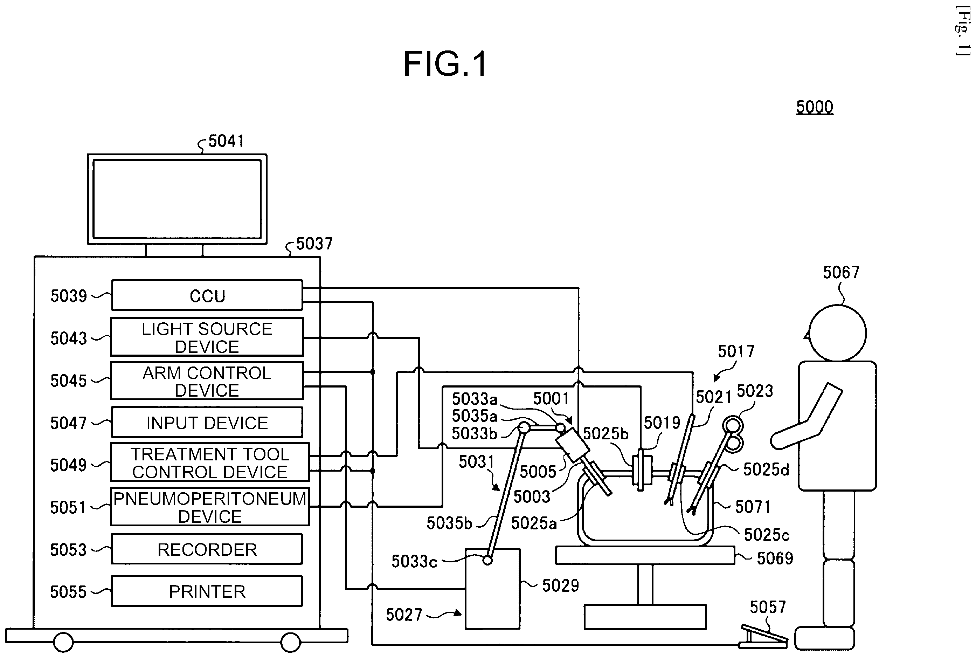

First, before description of details of the embodiment of the present disclosure, a schematic configuration of an endoscopic surgery system 5000 to which the technique according to the present disclosure can be applied will be described with reference to FIG. 1. FIG. 1 illustrates an example of a schematic configuration of the endoscopic surgery system 5000 to which the technique according to the present disclosure can be applied. FIG. 1 illustrates a surgeon (doctor) 5067 performing surgery on a patient 5071 on a patient bed 5069 by using the endoscopic surgery system 5000. As illustrated in FIG. 1, the endoscopic surgery system 5000 includes an endoscope 5001, other surgical tools 5017, a support arm device 5027 that supports the endoscope 5001, and a cart 5037 on which various types of devices for endoscopic surgery are mounted. Hereinafter, details of the endoscopic surgery system 5000 will be sequentially described.

### (Surgical tools 5017)

In endoscopic surgery, for example, a plurality of tubular perforating devices called trocars 5025a to 5025d are passed through an abdominal wall instead of cutting and opening the abdominal wall. Then, a lens barrel 5003 of the endoscope 5001 and other surgical tools 5017 are inserted into a body cavity of the patient 5071 through the trocars 5025a to 5025d. In the example illustrated in FIG. 1, a pneumoperitoneum tube 5019, an energy treatment tool 5021, and forceps 5023 are inserted into the body cavity of the patient 5071 as the other surgical tools 5017. The energy treatment tool 5021 is a treatment tool that, for example, cuts and peels tissue or seals a blood vessel by using a high-frequency current or ultrasonic vibration. However, the surgical tools 5017 illustrated in FIG. 1 are merely an example, and examples of the surgical tools 5017 include various surgical tools generally used in endoscopic surgery, such as tweezers and retractors.

### (Support arm device 5027)

The support arm device 5027 has an arm portion 5031 extending from a base portion 5029. In the example illustrated in FIG. 1, the arm portion 5031 is constituted by joint portions 5033a, 5033b, and 5033c and links 5035a and 5035b and is driven by control from an arm control device 5045. The endoscope 5001 is supported by the arm portion 5031 so that position and posture of the endoscope 5001 are controlled. As a result, the position of the endoscope 5001 can be fixed stably.

### (Endoscope 5001)

The endoscope 5001 is constituted by the lens barrel 5003 whose region having a predetermined length from a tip thereof is inserted into the body cavity of the patient 5071 and a camera head 5005 connected to a base end of the lens barrel 5003. Although a rigid scope having a rigid lens barrel 5003 is illustrated as the endoscope 5001 in the example illustrated in FIG. 1, the endoscope 5001 may be a flexible scope having a flexible lens barrel 5003 and is not limited in particular in the embodiment of the present disclosure.

The tip of the lens barrel 5003 is provided with an opening in which an objective lens is fitted. A light source device 5043 is connected to the endoscope 5001, and light generated by the light source device 5043 is guided to the tip of the lens barrel by a light guide extending inside the lens barrel 5003 so that an observation target in the body cavity of the patient 5071 is irradiated with the light through the objective lens. In the embodiment of the present disclosure, the endoscope 5001 may be a forward direct viewing endoscope or may be an oblique-viewing endoscope and is not limited in particular.

An optical system and a light receiving element are provided inside the camera head 5005, and reflected light (observation light) from the observation target is collected onto the light receiving element by the optical system. The observation light is photoelectrically converted by the light receiving element, and an electric signal corresponding to the observation light, that is, a pixel signal corresponding to the observation image is generated. The pixel signal is transmitted as RAW data to a camera control unit (CCU) 5039. The camera head 5005 is equipped with a function of adjusting a magnification and a focal length by appropriately driving the optical system.

The camera head 5005 may be provided with a plurality of light receiving elements in order to support, for example, stereoscopic viewing (3D display). In this case, a plurality of relay optical systems are provided inside the lens barrel 5003 in order to guide observation light to each of the plurality of light receiving elements.

### (Various devices mounted on cart)

First, a display device 5041 displays an image based on an image signal generated by performing image processing on a pixel signal by the CCU 5039 under control of the CCU 5039. In a case where the endoscope 5001 supports high-resolution shooting such as 4K (3840 pixels in a horizontal direction × 2160 pixels in a vertical direction) or 8K (7680 pixels in a horizontal direction × 4320 pixels in a vertical direction) and/or in a case where the endoscope 5001 supports 3D display, a display device capable of high-resolution display and/or 3D display is used as the display device 5041 accordingly. Further, a plurality of display devices 5041 having different resolutions and sizes may be provided depending on the application.

In addition, an image of a surgical site in the body cavity of the patient 5071 taken by the endoscope 5001 is displayed on the display device 5041. The surgeon 5067 can perform a procedure such as excising an affected area by using the energy treatment tool 5021 or the forceps 5023 while viewing the image of the surgical site displayed on the display device 5041 in real time. Note that the pneumoperitoneum tube 5019, the energy treatment tool 5021, and the forceps 5023 may be supported by the surgeon 5067, an assistant, or the like during surgery.

The CCU 5039 is constituted by a central processing unit (CPU), a graphics processing unit (GPU), and the like, and can collectively control operations of the endoscope 5001 and the display device 5041. Specifically, the CCU 5039 performs, on a pixel signal received from the camera head 5005, various kinds of image processing, such as development processing (demosaic processing), for displaying an image based on the pixel signal. Further, the CCU 5039 provides the display device 5041 with an image signal generated by performing the image processing. The CCU 5039 also transmits a control signal to the camera head 5005 to control driving of the camera head 5005. The control signal can include information about imaging conditions such as magnification and focal length.

The light source device 5043 is, for example, a light source such as a light emitting diode (LED), and supplies irradiation light for photographing a surgical site to the endoscope 5001.

The arm control device 5045 is, for example, a processor such as a CPU, and operates according to a predetermined program to control driving of the arm portion 5031 of the support arm device 5027 according to a predetermined control method.

An input device 5047 is an input interface to the endoscopic surgery system 5000.

The surgeon 5067 can enter various information and instructions to the endoscopic surgery system 5000 by using the input device 5047. For example, the surgeon 5067 enters various information related to surgery, such as physical information of a patient and information about a surgical procedure, by using the input device 5047. Further, for example, the surgeon 5067 can enter instructions such as an instruction to drive the arm portion 5031, an instruction to change imaging conditions (e.g., type of irradiation light, magnification, focal length) of the endoscope 5001, and an instruction to drive the energy treatment tool 5021 by using the input device 5047. The type of the input device 5047 is not limited, and the input device 5047 may be any of various known input devices. As the input device 5047, for example, a mouse, a keyboard, a touch panel, a switch, a foot switch 5057, a lever, and/or the like can be applied. For example, in a case where a touch panel is used as the input device 5047, the touch panel may be provided on a display surface of the display device 5041.

Alternatively, the input device 5047 may be a device worn by the surgeon 5067, such as a glasses-type wearable device or a head mounted display (HMD). In this case, various instructions are entered based on gestures and lines of sight of the surgeon 5067 detected by such a device. The input device 5047 can include a camera capable of detecting movement of the surgeon 5067, and various instructions may be entered based on gestures and lines of sight of the surgeon 5067 detected from an image taken by the camera. Further, the input device 5047 can include a microphone capable of picking up voice of the surgeon 5067, and various instructions may be entered based on voice through the microphone. In a case where the input device 5047 is configured to be able to receive various information in a non-contact manner as described above, a user (e.g., the surgeon 5067) belonging to a clean area can operate a device belonging to an unclean area in a non-contact manner. In addition, the surgeon 5067 can operate the device without taking his or her hand off a surgical tool in possession. This improves convenience of the surgeon 5067.

A treatment tool control device 5049 controls driving of the energy treatment tool 5021 for ablation of tissue, incision, sealing of blood vessels, and the like. A pneumoperitoneum device 5051 sends gas into the body cavity of the patient 5071 through the pneumoperitoneum tube 5019 for the purpose of securing a field of view of the endoscope 5001 and securing a surgeon's work space. A recorder 5053 is a device that can record various information related to surgery. A printer 5055 is a device capable of printing various information related to surgery in various formats such as a text, an image, and a graph.

### <1.2 Example of detailed configuration of support arm device 5027>

Further, an example of a detailed configuration of the support arm device 5027 will be described. The support arm device 5027 has the base portion 5029 as a base and the arm portion 5031 extending from the base portion 5029. The arm portion 5031 is constituted by a plurality of joint portions 5033a, 5033b, and 5033c and a plurality of links 5035a and 5035b connected by the joint portion 5033b in the example illustrated in FIG. 1, but the configuration of the arm portion 5031 is simplified in FIG. 1. Specifically, shapes, the numbers, and arrangement of the joint portions 5033a to 5033c and the links 5035a and 5035b, directions of rotation axes of the joint portions 5033a to 5033c, and the like are appropriately set so that the arm portion 5031 has a desired degree of freedom. For example, the arm portion 5031 may be configured to have a degree of freedom of 6 or more. This allows the endoscope 5001 to freely move within a movable range of the arm portion 5031, thereby allowing the lens barrel 5003 of the endoscope 5001 to be inserted into the body cavity of the patient 5071 from a desired direction.

Each of the joint portions 5033a to 5033c is provided with an actuator, and the joint portions 5033a to 5033c are configured to be rotatable around predetermined rotation axes by driving of the actuator. By controlling driving of the actuator by the arm control device 5045, rotation angles of the joint portions 5033a to 5033c are controlled, and thus driving of the arm portion 5031 is controlled. This can realize control of the position and posture of the endoscope 5001. The arm control device 5045 can control driving of the arm portion 5031 by any of various known control methods such as force control and position control.

For example, the position and posture of the endoscope 5001 may be controlled as follows. Specifically, the surgeon 5067 makes operation input as appropriate by using the input device 5047 (including the foot switch 5057), and driving of the arm portion 5031 is controlled as appropriate by the arm control device 5045 in response to the operation input. The arm portion 5031 may be operated by a master slave method. In this case, the arm portion 5031 (slave) can be remotely operated by the surgeon 5067 via the input device 5047 (master console) installed at a location away from an operating room or in the operating room.

In general, in endoscopic surgery, the endoscope 5001 is supported by a doctor called a scopist. On the other hand, in the embodiment of the present disclosure, use of the support arm device 5027 allows the position of the endoscope 5001 to be fixed with more certainty without human intervention, thereby making it possible to obtain an image of a surgical site stably and perform surgery smoothly.

The arm control device 5045 need not necessarily be provided on the cart 5037. Further, the arm control device 5045 need not necessarily be a single device. For example, the arm control device 5045 may be provided at each of the joint portions 5033a to 5033c of the arm portion 5031 of the support arm device 5027 or driving of the arm portion 5031 may be controlled by a plurality of arm control devices 5045 cooperating with each other.

### <1.3 Example of detailed configuration of light source device 5043>

Next, an example of a detailed configuration of the light source device 5043 will be described. The light source device 5043 supplies the endoscope 5001 with irradiation light for photographing a surgical site. The light source device 5043 is, for example, constituted by an LED, a laser light source, or a white light source combining an LED and a laser light source. In a case where a white light source is constituted by a combination of RGB laser light sources, an output intensity and an output timing of each color (each wavelength) can be controlled with high accuracy, and therefore white balance of a taken image can be adjusted in the light source device 5043. In this case, images corresponding to RGB can be taken in a time-divided manner by irradiating an observation target with laser light from the RGB laser light sources in a time-divided manner and controlling driving of the light receiving element of the camera head 5005 in synchronization with a timing of the irradiation. According to this method, a color image can be obtained without providing a color filter in the light receiving element.

Further, driving of the light source device 5043 may be controlled so that an intensity of output light is changed at predetermined time intervals. A high-dynamic-range image without blocked up shadows and blown out highlights can be generated by controlling driving of the light receiving element of the camera head 5005 in synchronization with a timing of the change of the intensity of the light to acquire images in a time-divided manner and synthesizing the images.

Further, the light source device 5043 may be configured to be able to supply light in a predetermined wavelength band corresponding to special light observation. In the special light observation, for example, narrow band imaging of photographing a predetermined issue such as a blood vessel in a superficial portion of a mucous membrane with high contrast by emitting light in a narrower band than irradiation light (i.e., white light) for normal observation by utilizing wavelength dependence of light absorption in body tissue. Alternatively, in the special light observation, fluorescence observation in which an image is obtained by fluorescence generated by irradiation with excitation light may be performed. In the fluorescence observation, for example, fluorescence from a body tissue may be observed by irradiating the body tissue with excitation light (autofluorescence imaging) or a fluorescence image may be obtained by locally injecting a reagent such as indocyanine green (ICG) into a body tissue and irradiating the body tissue with excitation light corresponding to a fluorescence wavelength of the reagent. The light source device 5043 may be configured to be capable of supplying such narrow band light corresponding to special light observation and/or excitation light.

### <1.4 Example of detailed configurations of camera head 5005 and CCU 5039>

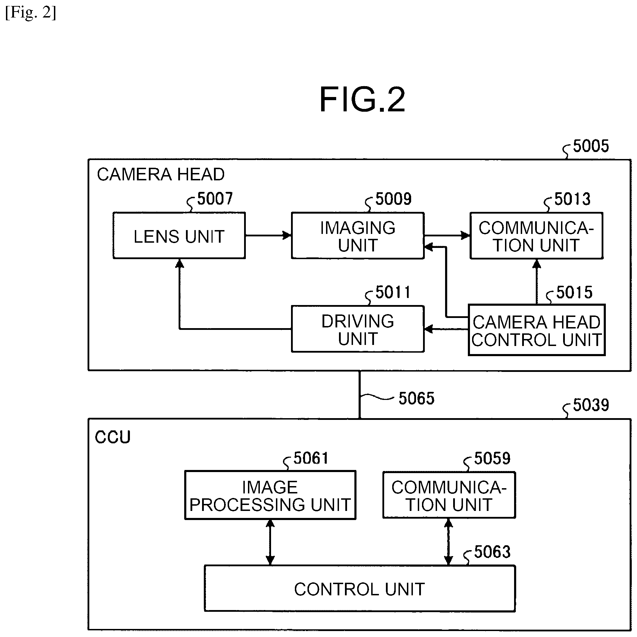

Next, an example of the detailed configurations of the camera head 5005 and the CCU 5039 will be described with reference to FIG. 2. FIG. 2 is a block diagram illustrating an example of functional configurations of the camera head 5005 and the CCU 5039 illustrated in FIG. 1.

More specifically, as illustrated in FIG. 2, the camera head 5005 has a lens unit 5007, an imaging unit 5009, a driving unit 5011, a communication unit 5013, and a camera head control unit 5015 as functions thereof. The CCU 5039 has a communication unit 5059, an image processing unit 5061, and a control unit 5063 as functions thereof. The camera head 5005 and the CCU 5039 are connected so as to be bidirectionally communicable with each other by a transmission cable 5065.

First, the functional configuration of the camera head 5005 will be described. The lens unit 5007 is an optical system provided at a connection portion with the lens barrel 5003. Observation light taken in from the tip of the lens barrel 5003 is guided to the camera head 5005 and incident on the lens unit 5007. The lens unit 5007 is constituted by combining a plurality of lenses including a zoom lens and a focus lens. Optical characteristics of the lens unit 5007 are adjusted so that observation light is collected on a light receiving surface of the light receiving element of the imaging unit 5009. Further, the zoom lens and the focus lens are configured so that positions thereof on an optical axis are movable in order to adjust magnification and focal point of a taken image.

The imaging unit 5009 is constituted by a light receiving element and is disposed in a stage following the lens unit 5007. The observation light that has passed through the lens unit 5007 is focused on the light receiving surface of the light receiving element, and a pixel signal corresponding to the observation image is generated by photoelectric conversion. The pixel signal generated by the imaging unit 5009 is provided to the communication unit 5013.

As the light receiving element constituting the imaging unit 5009, for example, a complementary metal oxide semiconductor (CMOS) type image sensor capable of color photographing having a Bayer array is used. As the light receiving element, for example, a light receiving element that can support a high resolution image of 4K or higher may be used. In a case where a high resolution image of a surgical site is obtained, the surgeon 5067 can grasp a state of the surgical site in more detail and proceed with surgery more smoothly.

Further, the light receiving element constituting the imaging unit 5009 may be configured to have a pair of light receiving elements for acquiring pixel signals for right and left eyes that support 3D display (stereo method). The 3D display enables the surgeon 5067 to more accurately grasp a depth of a living tissue in a surgical site and to grasp a distance to the living tissue. In a case where the imaging unit 5009 is a multi-plate type, a plurality of lens units 5007 may be provided corresponding to respective light receiving elements.

Further, the imaging unit 5009 need not necessarily be provided in the camera head 5005. For example, the imaging unit 5009 may be provided inside the lens barrel 5003 so as to immediately follow the objective lens.

The driving unit 5011 is constituted by an actuator and moves the zoom lens and the focus lens of the lens unit 5007 by a predetermined distance along the optical axis under control of the camera head control unit 5015. As a result, the magnification and focal point of the image taken by the imaging unit 5009 can be adjusted as appropriate.

The communication unit 5013 is constituted by a communication device for transmitting and receiving various information to and from the CCU 5039. The communication unit 5013 transmits a pixel signal obtained from the imaging unit 5009 as RAW data to the CCU 5039 through the transmission cable 5065. To display a low-latency image of a surgical site, the pixel signal may be transmitted by optical communication. This is because the surgeon 5067 performs surgery while observing a state of an affected area based on a taken image and therefore it is required that a moving image of the surgical site be displayed in real time to the extent possible for safer and more certain surgery. In a case where optical communication is performed, the communication unit 5013 is provided with a photoelectric conversion module that converts an electric signal into an optical signal. The pixel signal is converted into an optical signal by the photoelectric conversion module, and then transmitted to the CCU 5039 through the transmission cable 5065.

Further, the communication unit 5013 receives a control signal for controlling driving of the camera head 5005 from the CCU 5039. The control signal includes, for example, information concerning imaging conditions such as information designating a frame rate of a taken image, information designating an exposure value during imaging, and/or information designating magnification and focal point of the taken image. The communication unit 5013 supplies the received control signal to the camera head control unit 5015. The control signal from the CCU 5039 may also be transmitted by optical communication. In this case, the communication unit 5013 is provided with a photoelectric conversion module that converts an optical signal into an electric signal, and the control signal is converted into an electric signal by the photoelectric conversion module and is then supplied to the camera head control unit 5015.

The imaging conditions such as a frame rate, an exposure value, a magnification, and a focal point are automatically set by the control unit 5063 of the CCU 5039 based on an acquired pixel signal. That is, the endoscope 5001 is provided with an auto exposure (AE) function, an auto focus (AF) function, and an auto white balance (AWB) function.

The camera head control unit 5015 controls driving of the camera head 5005 based on the control signal from the CCU 5039 received via the communication unit 5013. For example, the camera head control unit 5015 controls driving of the light receiving element of the imaging unit 5009 based on the information designating a frame rate of a taken image and/or the information designating exposure during imaging. Further, for example, the camera head control unit 5015 causes the driving unit 5011 to appropriately move the zoom lens and the focus lens of the lens unit 5007 based on the information designating magnification and focal point of a taken image. The camera head control unit 5015 may further have a function of storing information for identifying the lens barrel 5003 and the camera head 5005.

By arranging the members such as the lens unit 5007 and the imaging unit 5009 in a sealed structure having high airtightness and waterproofness, the camera head 5005 can be made resistant to autoclave sterilization.

Next, the functional configuration of the CCU 5039 will be described. The communication unit 5059 is constituted by a communication device for transmitting and receiving various information to and from the camera head 5005. The communication unit 5059 receives a pixel signal transmitted from the camera head 5005 through the transmission cable 5065. As described above, the pixel signal can be suitably transmitted by optical communication. In this case, to support the optical communication, the communication unit 5059 is provided with a photoelectric conversion module that converts an optical signal into an electric signal. The communication unit 5059 supplies a pixel signal converted into an electric signal to the image processing unit 5061.

Further, the communication unit 5059 transmits a control signal for controlling driving of the camera head 5005 to the camera head 5005. The control signal may also be transmitted by optical communication.

The image processing unit 5061 performs various image processing on a pixel signal which is RAW data transmitted from the camera head 5005. Examples of the image processing include various kinds of known signal processing such as development processing, high image quality processing (band enhancement processing, super-resolution processing, noise reduction (NR) processing, camera shake correction processing, and/or the like) and/or enlargement processing (electronic zoom processing). In addition, the image processing unit 5061 performs detection processing for AE, AF, and AWB on a pixel signal.

The image processing unit 5061 is constituted by a processor such as a CPU or a GPU. The processor operates in accordance with a predetermined program, and thereby the above image processing and detection processing can be performed. In a case where the image processing unit 5061 is constituted by a plurality of GPUs, the image processing unit 5061 appropriately divides information related to a pixel signal and performs image processing in parallel by the plurality of GPUs.

The control unit 5063 performs various controls regarding imaging of a surgical site by the endoscope 5001 and display of an image thus taken. For example, the control unit 5063 generates a control signal for controlling driving of the camera head 5005. In a case where imaging conditions have been entered by the surgeon 5067, the control unit 5063 generates a control signal based on the entry of the surgeon 5067. Alternatively, in a case where the endoscope 5001 is provided with an AE function, an AF function, and an AWB function, the control unit 5063 calculates optimum exposure value, focal length, and white balance in accordance with a result of the detection processing by the image processing unit 5061 and generate a control signal.

Further, the control unit 5063 causes the display device 5041 to display an image of a surgical site based on an image signal generated by performing the image processing by the image processing unit 5061. The control unit 5063 recognizes various objects in the surgical site image by using various image recognition techniques. For example, the control unit 5063 can recognize surgical tools such as forceps, a specific biological part, bleeding, mist during use of the energy treatment tool 5021, and the like by detecting a shape, a color, and the like of an edge of an object included in the surgical site image. When displaying an image of the surgical site on the display device 5041, the control unit 5063 superimposes various surgery support information on the image of the surgical site by using a result of the recognition. By superimposing the surgery support information and presenting the surgery support information to the surgeon 5067, it becomes possible to proceed with the surgery more safely and surely.

The transmission cable 5065 that connects the camera head 5005 and the CCU 5039 is an electric signal cable that supports electrical signal communication, an optical fiber that supports optical communication, or a composite cable thereof.

Although communication is performed by wire by using the transmission cable 5065 in the example illustrated in FIG. 2, the communication between the camera head 5005 and the CCU 5039 may be performed wirelessly. In a case where the communication between the camera head 5005 and the CCU 5039 is performed wirelessly, it is not necessary to dispose the transmission cable 5065 in the operating room. This can prevent a situation where movement of medical staff in the operating room is hindered by the transmission cable 5065.

### <1.5 Example of configuration of endoscope 5001>

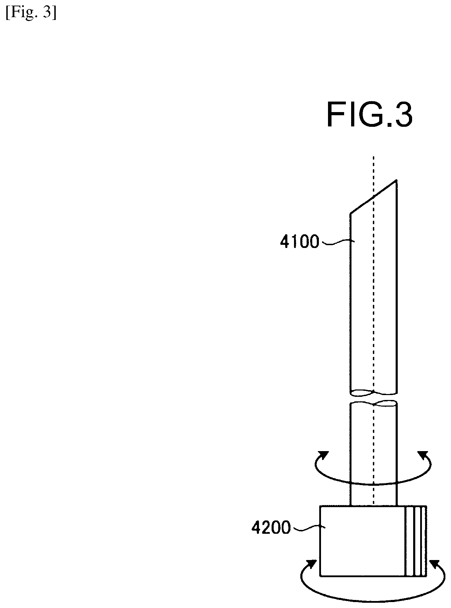

Next, a basic configuration of an oblique-viewing endoscope will be described as an example of the endoscope 5001 with reference to FIG. 3. FIG. 3 is a schematic view illustrating a configuration of an oblique-viewing endoscope 4100 according to an embodiment of the present disclosure.

Specifically, as illustrated in FIG. 3, the oblique-viewing endoscope 4100 is attached to a tip of a camera head 4200. The oblique-viewing endoscope 4100 corresponds to the lens barrel 5003 described in FIGS. 1 and 2, and a camera head 4200 corresponds to the camera head 5005 described in FIGS. 1 and 2. The oblique-viewing endoscope 4100 and the camera head 4200 can rotate independently of each other. An actuator is provided between the oblique-viewing endoscope 4100 and the camera head 4200 as with the joint portions 5033a, 5033b, and 5033c, and the oblique-viewing endoscope 4100 rotates with respect to the camera head 4200 by driving of the actuator.

The oblique-viewing endoscope 4100 is supported by the support arm device 5027. The support arm device 5027 has a function of holding the oblique-viewing endoscope 4100 instead of the scopist and moving the oblique-viewing endoscope 4100 so that a desired site can be observed by a surgeon or assistant's operation.

In the embodiment of the present disclosure, the endoscope 5001 is not limited to the oblique-viewing endoscope 4100. For example, the endoscope 5001 may be a forward direct viewing endoscope that captures a front side of the tip of the endoscope, and may have a function of cutting out an image from a wide-angle image captured by the endoscope (wide angle/cutting out function). Further, for example, the endoscope 5001 may be an endoscope with a tip bending function capable of changing a field of view by freely bending the tip of the endoscope according to an operation of the surgeon 5067. Further, for example, the endoscope 5001 may be an endoscope (with another direction concurrent photographing function that has a plurality of camera units having different fields of view at the tip of the endoscope and can obtain different images by using cameras.

An example of the endoscopic surgery system 5000 to which the technique according to the present disclosure can be applied has been described above. Although the endoscopic surgery system 5000 has been described as an example, a system to which the technique according to the present disclosure can be applied is not limited to this example. For example, the technique according to the present disclosure may be applied to a microscopic surgery system.

### <<2. Medical observation system>>

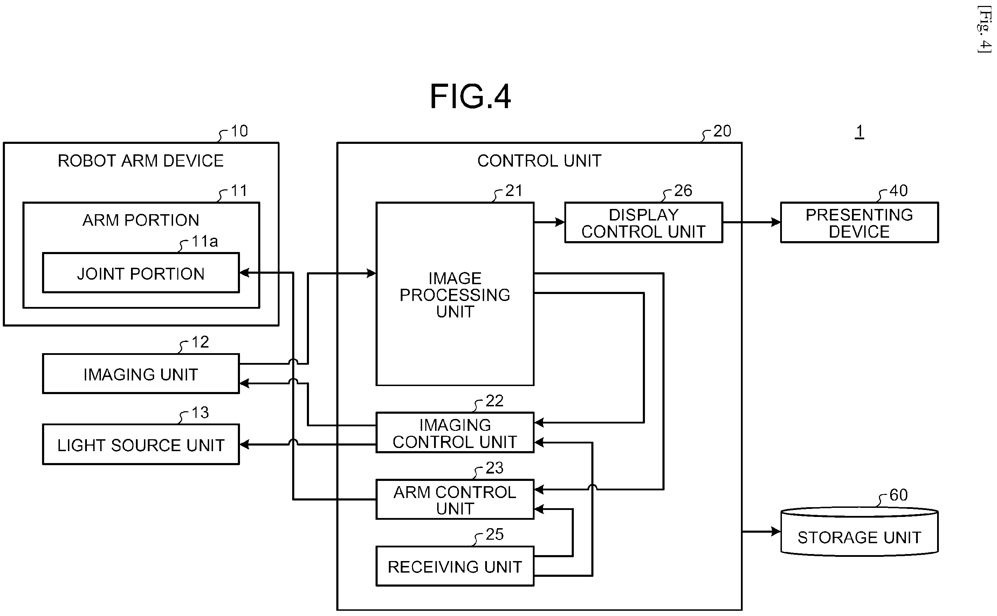

Further, a configuration of a medical observation system 1 according to the embodiment of the present disclosure, which can be combined with the endoscopic surgery system 5000 described above, will be described with reference to FIG. 4. FIG. 4 is a block diagram illustrating an example of the configuration of the medical observation system 1 according to the embodiment of the present disclosure. As illustrated in FIG. 4, the medical observation system 1 mainly includes a robot arm device 10, an imaging unit 12, a light source unit 13, a control unit 20, a presenting device 40, and a storage unit 60. Functional units included in the medical observation system 1 will be described below.

First, before explaining details of the configuration of the medical observation system 1, an outline of processing of the medical observation system 1 will be described. In the medical observation system 1, first, an abdominal cavity of a patient is imaged to recognize an environment in the abdominal cavity, and the robot arm device 10 can be driven based on the recognition result of the environment in the abdominal cavity.

### (Robot arm device 10)

The robot arm device 10 has an arm portion 11 (multi-joint arm) which is a multi-link structure constituted by a plurality of joint portions and a plurality of links, and controls a position and a posture of a tip unit provided at a tip of the arm portion by driving the arm portion within a movable range. The robot arm device 10 corresponds to the support arm device 5027 illustrated in FIG. 1.

The robot arm device 10 can have, for example, the CCU 5039 illustrated in FIG. 2, an electronic cutting control unit that cuts out a predetermined region from an image of a target object received from the CCU 5039 and outputs the predetermined region to a GUI generation unit described later, a posture control unit that controls a position and a posture of the arm portion 11, and the GUI generation unit that generates image data by performing various kinds of processing on the image cut out by the electronic cutting control unit.

In the robot arm device 10 according to the embodiment of the present disclosure, an electronic degree of freedom of changing a line of sight by cutting out a taken image (wide angle/cutting out function) and a degree of freedom realized by the actuator of the arm portion 11 are all treated as a degree of freedom of a robot. This makes it possible to realize motion control in which the electronic degree of freedom of changing the line of sight and the degree of freedom of the joints realized by the actuator are linked.

Specifically, the arm portion 11 is a multi-link structure constituted by a plurality of joint portions and a plurality of links, and driving thereof is controlled by control from an arm control unit 23 described later. The arm portion 11 corresponds to the arm portion 5031 illustrated in FIG. 1. In FIG. 4, one joint portion 11a is illustrated as a representative of the plurality of joint portions. Specifically, the joint portion 11a rotatably connects the links with each other in the arm portion 11, and rotational driving of the joint portion 11a is controlled by control from the arm control unit 23 to drive the arm portion 11. In the present embodiment, information on the position and posture of the arm portion 11 can be obtained based on joint angles and link lengths of a joint portions 5033, a link 5035 (See fig.1), and the like included in the arm portion 11. In the present embodiment, the arm portion 11 may have a motion sensor, examples of which include an acceleration sensor, a gyro sensor, and a geomagnetic sensor, in order to obtain information on the position and posture of the arm portion 11.

### (Imaging unit 12)

The imaging unit (medical observation device) 12 is provided at the tip of the arm portion (medical arm) 11 and takes images of various imaging target objects. That is, the arm portion 11 supports the imaging unit 12. As described above, the imaging unit 12 may be, for example, the oblique-viewing endoscope 4100, a forward direct viewing endoscope with a wide angle/cutting out function, an endoscope with a tip bending function, or an endoscope with other direction concurrent photographing function or may be a microscope, and is not limited in particular.

Further, the imaging unit 12 takes, for example, a surgical field image including various medical instruments, organs, and the like in an abdominal cavity of a patient. Specifically, the imaging unit 12 is a camera or the like capable of photographing a target in a moving image format or a still image format. More specifically, the imaging unit 12 is a wide-angle camera constituted by a wide-angle optical system. For example, an angle of view of the imaging unit 12 according to the present embodiment may be 140°, whereas an angle of view of a normal endoscope is about 80°. The angle of view of the imaging unit 12 may be smaller than 140° or may be 140° or more as long as the angle of view of the imaging unit 12 exceeds 80°. The imaging unit 12 transmits an electric signal (pixel signal) corresponding to a taken image to the control unit 20. Further, the arm portion 11 may support a medical instrument such as the forceps 5023.

Further, in the embodiment of the present disclosure, the imaging unit 12 may be a stereo-type endoscope (stereo endoscope) capable of measuring a distance. Alternatively, in the present embodiment, a depth sensor (distance measuring device) may be provided separately from the imaging unit 12. In this case, the imaging unit 12 can be a monocular endoscope. Specifically, the depth sensor can be, for example, a sensor that measures a distance by using a Time of Flight (ToF) method in which a distance is measured by using a return time of reflection of pulsed light from a subject or a structured light method for radiating grid-like pattern light and measuring a distance based on distortion of the pattern. Alternatively, in the present embodiment, the depth sensor may be provided in the imaging unit 12 itself. In this case, the imaging unit 12 can measure a distance by the ToF method at the same time as imaging. Specifically, the imaging unit 12 includes a plurality of light receiving elements, and can generate an image or calculate distance information based on pixel signals obtained from the light receiving elements.

### (Light source unit 13)

The light source unit 13 irradiates an object to be imaged by the imaging unit 12 with light. The light source unit 13 can be realized, for example, by a light emitting diode (LED) for a wide-angle lens. The light source unit 13 may be, for example, a combination of a normal LED and a lens to diffuse light. Further, the light source unit 13 may be configured such that light transmitted by an optical fiber (light guide) is diffused (widened) by a lens. Further, the light source unit 13 may widen an irradiation range by directing an optical fiber itself in a plurality of directions.

### (Control unit 20)

The control unit 20 is, for example, realized in a manner such that a program (for example, a program according to the embodiment of the present disclosure) stored in the storage unit 60 described later is executed by a central processing unit (CPU), a micro processing unit (MPU), or the like while using a random access memory (RAM) or the like as a work area. Further, the control unit 20 is a controller and may be realized, for example, by an integrated circuit such as an application specific integrated circuit (ASIC) or a field programmable gate array (FPGA). Specifically, the control unit 20 mainly includes an image processing unit 21, an imaging control unit 22, the arm control unit 23, a receiving unit 25, and a display control unit 26.

The image processing unit 21 executes various kinds of processing on an imaging target imaged by the imaging unit 12. Specifically, the image processing unit 21 acquires an image of an imaging target imaged by the imaging unit 12 and generates various images based on the image taken by the imaging unit 12. Specifically, the image processing unit 21 can generate an image by cutting out and enlarging a display target region of an image taken by the imaging unit 12. In this case, the image processing unit 21 may change a position to be cut out in accordance with, for example, the position and posture of the arm portion 11.

The imaging control unit 22 controls the imaging unit 12. The imaging control unit 22 controls, for example, the imaging unit 12 to image a surgical field. The imaging control unit 22 controls, for example, a magnification of the imaging unit 12. Further, the imaging control unit 22 may control the magnification of the imaging unit 12, for example, based on information entered by the surgeon 5067 received by the receiving unit 25.

Further, the imaging control unit 22 controls the light source unit 13. For example, the imaging control unit 22 controls brightness of the light source unit 13 when the imaging unit 12 images a surgical field. The imaging control unit 22 controls the brightness of the light source unit 13, for example, based on information entered by the surgeon 5067 received by the receiving unit 25.

The arm control unit 23 controls the robot arm device 10 in an integrated manner and controls driving of the arm portion 11. Specifically, the arm control unit 23 controls driving of the arm portion 11 by controlling driving of the joint portion 11a. More specifically, the arm control unit 23 controls a rotational speed of a motor in the actuator of the joint portion 11a by controlling an amount of current supplied to the motor and controls a rotation angle and generated torque in the joint portion 11a.

The arm control unit 23 can autonomously control the position and posture of the arm portion 11, for example, based on a learning model described later. For example, the arm control unit 23 autonomously controls the position and the posture of the arm portion 11 by using a learning model (learned model) obtained by performing machine learning by using, as learning data, various data (external input information) such as information entered by the surgeon 5067 received by the receiving unit 25 and an image obtained by the imaging unit 12. In this case, the arm control unit 23 may control the position and the posture of the arm portion 11, for example, by driving the joint portion 11a of the arm portion 11 so as to avoid a medical instrument that blocks a field of view of the surgeon 5067.

The arm control unit 23 can autonomously control the position and posture of the arm portion 11, for example, based on a learning model described later. The arm control unit 23 autonomously controls the position and the posture of the arm portion 11, for example, by referring to a learning model obtained by machine learning of learning data based on various data (external input information) such as information entered by the surgeon 5067 received by the receiving unit 25 and an image obtained by the imaging unit 12. In this case, the arm control unit 23 may control the position and the posture of the arm portion 11, for example, by driving the joint portion 11a of the arm portion 11 so as to avoid a medical instrument that blocks a field of view of the surgeon 5067.

The learning model may be generated, for example, based on data on other surgery. The data on surgery may include, for example, information about a medical instrument used in the surgery. The information about a medical instrument may include, for example, image data generated by the medical instrument and information about an operation of the medical instrument. In the embodiment of the present disclosure, discrimination accuracy can be improved by using a learning model generated based on image data obtained by various medical instruments and information about operations of various medical instruments.

Specifically, the learning data may include, for example, sensing data obtained from at least one of a stereo sensor, a depth sensor, and a motion sensor. More specifically, the learning data may include information on a surgical field environment including at least one of a position, a posture, a type, and movement of a medical instrument and a position, a posture, and a type of an organ obtained from at least one of a stereo sensor, a depth sensor, and a motion sensor.

The data on surgery used to generate a learned model may include information about the arm portion 11. The information about the arm portion 11 may include, for example, information about a state of the joint portion 11a of the arm portion 11. The information about the state of the joint portion 11a of the arm portion 11 may include, for example, various information such as a position, a posture, and movement of the joint portion of the arm portion 11. In the embodiment of the present disclosure, discrimination accuracy can be improved by using a learning model generated based on various information of the arm portion 11.

Further, the information about the arm portion 11 may include information about a medical instrument held by the arm portion 11. The information about the medical instrument may include, for example, at least one of a type of medical instrument, and position information and posture information of the medical instrument.

Further, in the embodiment of the present disclosure, the control unit 20 may have a function of generating a learning model. In this case, the control unit 20 generates a learning model and stores the generated learning model in the storage unit 60 described later.

The learning model used in the embodiment of the present disclosure is generated by learning features and the like of various input information used when classifying the input information and performing processing according to a classification result. The learning model may be realized by a deep neural network (DNN) or the like, which is a multi-layer neural network having a plurality of nodes including an input layer, a plurality of intermediate layers (hidden layers), and an output layer. However, the embodiment of the present disclosure is not limited to this. For example, to generate a learning model, first, various input information is input via an input layer, and processing such as extraction of features of the input information is performed in a plurality of intermediate layers connected in series. Next, various processing results such as classification results based on information output by the intermediate layers are output as output information corresponding to the input information via an output layer. In this way, a learning model can be generated.

The control unit 20 may generate a learning model for various kinds of surgery or may hold a predetermined model. The control unit 20 generates a learning model, for example, from learning data including a treatment status including information on treatment by a surgeon and endoscopic investigation data on a camera operation by a scopist. The control unit 20 generates a learning model, for example, by using, as learning data, positions and postures of a medical instrument and an endoscope measured by a tracking device. The control unit 20 generates a learning model, for example, by using, as learning data, an endoscope image including a depth and movement of each object photographed by a stereo endoscope and a type of medical instrument.

The control unit 20 may generate a learning model by using data about various surgical robots as learning data. The control unit 20 generates a learning model, for example, by using, as learning data, various operation information for a surgical robot by a doctor and a scopist. The control unit 20 may generate a learning model, for example, by using, as learning data, treatment using the support arm device 5027 illustrated in FIG. 1.

The receiving unit 25 receives an input operation by the surgeon 5067 and various input information from another device (sensor) and outputs such information to the imaging control unit 22 and the arm control unit 23. Details of the input information will be described later.

The display control unit 26 causes the presenting device 40, which will be described later, to display various images. For example, the display control unit 26 causes the presenting device 40 to display an image acquired from the imaging unit 12.

### (Presenting device 40)

The presenting device 40 displays various images. The presenting device 40 displays, for example, an image taken by the imaging unit 12. The presenting device 40 can be, for example, a display such as a liquid crystal display (LCD) or an organic EL (Organic Electro-Luminescence) display.

### (Storage unit 60)

The storage unit 60 stores various types of information. The storage unit 60 stores, for example, a learning model. The storage unit 60 is realized, for example, by a semiconductor memory element such as a random access memory (RAM) or a flash memory or a storage device such as a hard disk or an optical disk.

### <<3. Background leading to embodiment of present disclosure>>

In recent years, in the medical observation system 1, techniques for allowing the robot arm device 10 to autonomously operate have been developed. For example, the autonomous operation of the robot arm device 10 in the medical observation system 1 can be divided into various levels. Examples of the levels include (1) a level at which the surgeon (doctor) 5067 is guided by the system and (2) a level at which some operations (tasks) in surgery such as movement of the position of the imaging unit 12 performed by a scopist and suturation of a surgical site are executed autonomously by the system. Examples of the levels further include (3) a level at which operations during surgery are automatically generated by the system and the robot arm device 10 performs an operation selected by a doctor from among the operations automatically generated. In the future, (4,5) a level at which the robot arm device 10 performs all tasks in surgery under supervision of a doctor or without supervision of a doctor is also possible.

The embodiment of the present disclosure described below discuss, as an example, a case where the robot arm device 10 autonomously executes a task of moving the position of the imaging unit 12 instead of a scopist, and the surgeon 5067 performs surgery directly or by remote control while referring to an image taken by the imaging unit 12 thus moved.

For the autonomous operation of the robot arm device 10, it is required to generate autonomous operation control information (for example, an autonomous control target value) for the autonomous operation in advance. Therefore, autonomous operation control information is generated in advance based on actual body internal environment information (for example, a state of an affected area or the like) by referring to a learning model obtained by machine learning of a body internal environment information (for example, a three-dimensional structure in an abdominal cavity, an organ state, an affected area state) and a corresponding surgical operation of the surgeon 5067, an operation rule, and the like. However, since a body shape, organ forms, organ positions, and the like vary from one patient to another, it is practically difficult to perform machine learning of all internal environment information in advance, and presence of an unknown body internal environment that cannot be guaranteed by the above learning model and the like is inevitable. Therefore, for example, in surgery under an environment in which the imaging unit 12 (for example, the endoscope 5001) moves in accordance with the autonomous operation control information, if there is a body internal environment that is not guaranteed by the autonomous operation control information, there are concerns about misalignment of the field of view of the imaging unit 12 with respect to an forceps operation and interference between the imaging unit 12 and a non-target object (organ or tissue) (the field of view of the imaging unit 12 is blocked by the non-target object or the imaging unit 12 itself collides with an object). It is therefore difficult to carry out the surgery suitably and smoothly.

One measure is to acquire information on the unknown body internal environment, reconstruct a learning model, and generate autonomous operation control information by using the reconstructed learning model. However, the reconstruction takes a certain preparation time, and therefore it is difficult to generate a learning model and autonomous operation control information in real time. Further, even if the learning model and the autonomous operation control information can be generated in real time, movement of the imaging unit 12 and the like are delayed with respect to a forceps operation of the surgeon 5067. To cope with such a delay, it is conceivable that the surgeon 5067 performs the forceps operation while predicting a change in an image taken by the imaging unit 12. However, this involves difficult prediction, and therefore it is still difficult to carry out surgery suitably and smoothly.

In view of the above circumstances, an embodiment of the present disclosure can generate autonomous operation control information for the robot arm device 10 suitable for various body internal environments in real time. In an embodiment of the present disclosure, autonomous operation control information suitable for various body internal environments can be generated in real time by correcting autonomous operation control information generated based on various data (external input information) based on a result of operation simulation of the robot arm device 10 in an actual body internal environment and a result of operation simulation of the robot arm device 10 in an ideal body internal environment, i.e., a stored or reference body internal environment. As used herein, "ideal" refers to prerecorded or stored data to be used as a reference. The surgeon 5067 can carry out surgery appropriately and smoothly with assistance from the robot arm device 10 controlled in accordance with such autonomous operation control information. Details of the embodiment of the present disclosure will be sequentially described below.

### <<4. Embodiment>>

The embodiment of the present disclosure can be mainly divided into two stages: a stage in which a learning model or the like used to generate autonomous operation control information is generated in advance (a learning model generation stage) and a stage in which autonomous operation is performed based on the learning model or the like thus generated (autonomous operation execution stage). First, the learning model generation stage according to the present embodiment will be described.

### <4.1 Example of detailed configuration of learning model generation unit 100>

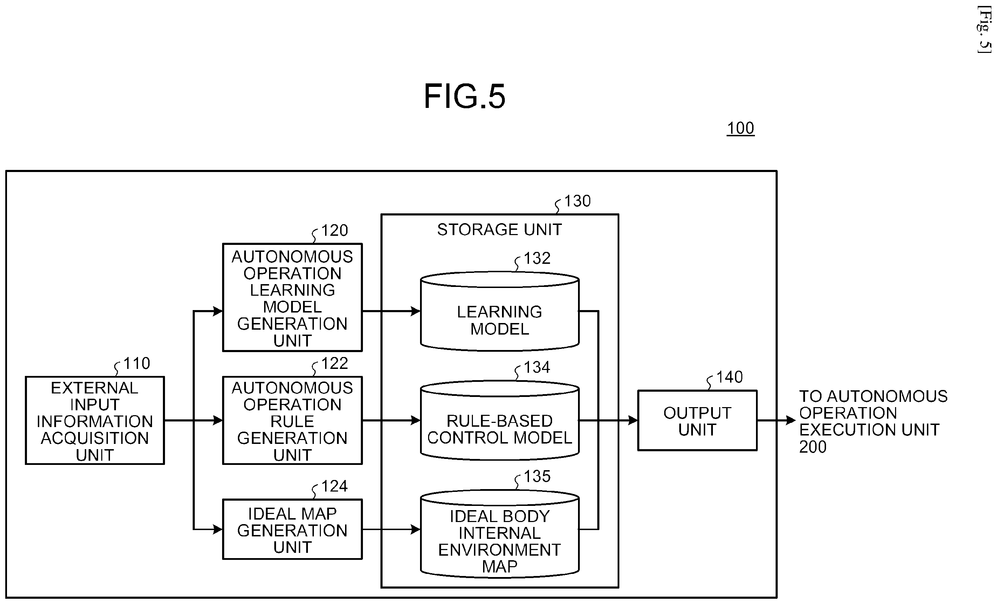

First, an example of a detailed configuration of a learning model generation unit 100 according to the embodiment of the present disclosure will be described with reference to FIG. 5. FIG. 5 is a block diagram illustrating an example of the configuration of the learning model generation unit 100 according to the embodiment of the present disclosure. The learning model generation unit 100 according to the present embodiment can generate a learning model 132, a rule-based control model 134, and an ideal (reference) body internal environment map 135 used to generate autonomous operation control information (specifically, an autonomous operation target value). The learning model generation unit 100 may be a device integrated with the robot arm device 10 or the control unit 20 illustrated in FIG. 4, may be a separate device, or may be a device that is provided on the cloud and is communicably connected to the robot arm device 10 and the control unit 20.

Specifically, as illustrated in FIG. 5, the learning model generation unit 100 mainly includes an external input information acquisition unit 110, an autonomous operation learning model generation unit 120, an autonomous operation rule generation unit 122, an ideal map generation unit 124, a storage unit 130, and an output unit 140. Details of the functional units of the learning model generation unit 100 will be sequentially described below.

### (External input information acquisition unit 110)

The external input information acquisition unit 110 acquires various data (external input information) and outputs the data to the autonomous operation learning model generation unit 120, the autonomous operation rule generation unit 122, and the ideal map generation unit 124. In the present embodiment, examples of the various data include tip position/posture data of forceps held in both left and right hands in ideally performed surgery or reference surgery that is prerecorded or stored(position information and posture information of a medical instrument in an actual environment), tip position/posture data of the imaging unit 12 (specifically, the endoscope 5001) (position information and posture information of a medical arm in an actual environment), an image taken by the imaging unit 12 (an image of an actual environment taken by a medical observation device), and sensing data from a distance measuring sensor (e.g., a stereo-type endoscope, a depth sensor). Specifically, in the present embodiment, the tip position/posture data may be acquired, for example, by using sensing data from a motion sensor provided on the arm portion 11 or a distance measuring sensor (a stereo-type endoscope (stereo camera), a structured light sensor, a ToF sensor) described above. In the present embodiment, information on the tip position/ posture data may be expressed as an absolute coordinate value or may be expressed as a relative coordinate value from a predetermined reference point, and is not limited in particular.

### (Autonomous operation learning model generation unit 120)

The autonomous operation learning model generation unit 120 generates the learning model 132 that outputs the position/posture of the imaging unit 12 with respect to the position/posture of forceps held by both left and right hands by performing machine learning using, as teacher data, a pair of tip position/posture data of forceps held by both left and right hands and tip position/posture data of the imaging unit 12 that are output from the external input information acquisition unit 110. In the present embodiment, the learning model 132 for realizing autonomous operation of the arm portion 11 and the like of the robot arm device 10 can be generated by performing machine learning using a pair of tip position/posture data of a large number of forceps held by both left and right hands and tip position/posture data of the imaging unit 12 in ideally performed surgery.

Alternatively, the autonomous operation learning model generation unit 120 can generate the learning model 132 that outputs visual field information including information such as a position of a gazing point located at a center of a field of view of the imaging unit 12, a distance from the imaging unit 12 to the gazing point, and a visual field direction to the gazing point with respect to any tip position/posture of the imaging unit 12 by performing machine learning using, as teacher data, a pair of an image taken by the imaging unit 12 and tip position/posture data of the imaging unit 12. The learning model 132 generated by the autonomous operation learning model generation unit 120 is stored in the storage unit 130, which will be described later.

### (Autonomous operation rule generation unit 122)

The autonomous operation rule generation unit 122 can generate the rule-based control model 134 that defines a control rule (e.g., permissible distance and positional relationship from and with an organ, permissible distance and positional relationship from and with forceps) permitted during autonomous operation of the arm portion 11 and the like of the robot arm device 10 by analyzing plural data collected in ideally performed surgery. The rule-based control model 134 generated by the autonomous operation rule generation unit 122 is stored in the storage unit 130, which will be described later.

Specifically, the autonomous operation rule generation unit 122 generates the rule-based control model 134, for example, by extracting a rule of position/posture of the imaging unit 12 with respect to any position/posture of forceps held by both left and right hands from a pair of tip position/posture data of forceps held by both left and right hands and tip position/posture data of the imaging unit 12 that are output from the external input information acquisition unit 110. Alternatively, the autonomous operation rule generation unit 122 generates the rule-based control model 134, for example, by extracting a rule of visual field information of the imaging unit 12 with respect to any image taken by the imaging unit 12 from a pair of an image taken by the imaging unit 12 and tip position/posture data of the imaging unit 12.

### (Ideal map generation unit 124)

The ideal map generation unit 124 generates a plurality of body internal environment maps based on various data used for generation of the learning model 132 and the rule-based control model 134. Three-dimensional map information (body internal environment map) in a body that realizes operation (also referred to as scope work) of the imaging unit 12 (specifically, the arm portion 11) that is closest to the ideal is referred to as an "ideal body internal environment map" or "reference body internal environment map". Furthermore, a body internal environment that realizes scope work closest to the ideal (reference) is referred to as an ideal (reference) environment or ideal (reference) body internal environment. The ideal body internal environment map 135 generated by the ideal map generation unit 124 is stored in the storage unit 130, which will be described later, and used in simulation that will be described later. In the present embodiment, the ideal body internal environment map 135 need not be limited to one, and there may be a plurality of ideal body internal environment maps 135. For example, a simulation execution environment may be, for example, updated every scene, and one ideal body internal environment map 135 may be selected from among the plurality of ideal body internal environment maps 135 in accordance with a function of the updated simulator. In this specification, "scope work close to the ideal" refers to scope work that can provide an image displayed at an appropriate magnification so that the surgeon 5067 can visually recognize peripheral organs and tissues and tips of forceps to perform a procedure while keeping a surgical site (a portion to be treated such as an organ or tissues) and forceps operating portions (portions located at the tips of the forceps where tissues are, for example, held) within a field of view and tracking movement of the tips of the forceps during insertion and removal of the forceps and capturing these at a center of a screen. That is, in the present specification, "scope work close to the ideal" can be rephrased as "appropriate scope work" capable of providing the above-mentioned image or as "readily perceivable scope work". In the present specification, the "forceps operation" may include not only excision and gripping operation by opening and closing of tips of the forceps, but also movement of the tips of the forceps such as change of positions of the tips of the forceps and insertion and removal. Further, in the present specification, the "ideal body internal environment map" is information of one map pattern by which the learning model can realize ideal scope work. In other words, the "ideal body internal environment map" refers to not mere 3D map information in a body but a known 3D map in a body that can realize scope work close to the ideal by using a learning model and a rule-based control model stored in a database.

Specifically, the ideal map generation unit 124 generates the ideal body internal environment map 135 from position information of an organ extracted from an image and position/posture information of the imaging unit 12 based on a pair of an image taken by the imaging unit 12 and the tip position/posture data of the imaging unit 12, for example, by using simultaneous localization and mapping (SLAM). Alternatively, the ideal map generation unit 124 generates the ideal body internal environment map 135 from position information of an organ extracted from an image and position/posture information of the imaging unit 12 based on a pair of an image taken by the imaging unit 12 and sensing data from a distance measuring sensor (e.g., a stereo endoscope, a depth sensor) by using SLAM. The ideal body internal environment map 135 may be a metric map using an expression method such as a grid, a boxel, or a point group.

### (Storage unit 130)

The storage unit 130 stores therein the learning model 132, the rule-based control model 134, and the ideal body internal environment map 135 described above. The storage unit 130 is realized, for example, by a semiconductor memory element such as a RAM or a flash memory or a storage device such as a hard disk or an optical disk.

### (Output unit 140)

The output unit 140 can output the learning model 132, the rule-based control model 134, and the ideal body internal environment map 135 described above to the autonomous operation execution unit 200, which will be described later.

In the present embodiment, the detailed configuration of the learning model generation unit 100 is not limited to the configuration illustrated in FIG. 5.

### <4.2 Control method in learning model generation stage>

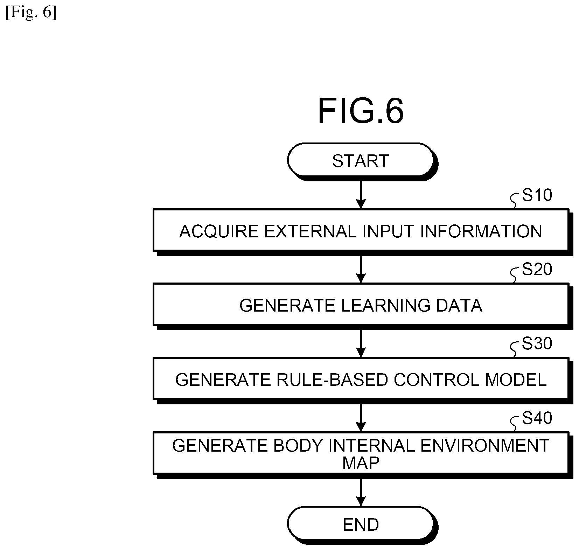

Next, a control method in the learning model generation stage according to the present embodiment will be described with reference to FIG. 6. FIG. 6 is a flowchart of the control method in the learning model generation stage according to the present embodiment. As shown in FIG. 6, the control method in the learning model generation stage according to the present embodiment can mainly include steps from step S10 to step S40. An outline of each of these steps according to the present embodiment will be described below.

First, the external input information acquisition unit 110 of the learning model generation unit 100 acquires various data (external input information) collected in ideally performed surgery (step S10). Next, the autonomous operation learning model generation unit 120 generates the learning model 132 by performing machine learning using the various data as teacher data and outputs the learning model 132 to the storage unit 130 and the autonomous operation execution unit 200 (step S20). Further, the autonomous operation rule generation unit 122 generates the rule-based control model 134 by analyzing the various data (external input information) and outputs the rule-based control model 134 to the storage unit 130 and the autonomous operation execution unit 200 (step S30). Then, the ideal map generation unit 124 generates a body internal environment map based on the various data and outputs the body internal environment map to the storage unit 130 and the autonomous operation execution unit 200 (step S40).

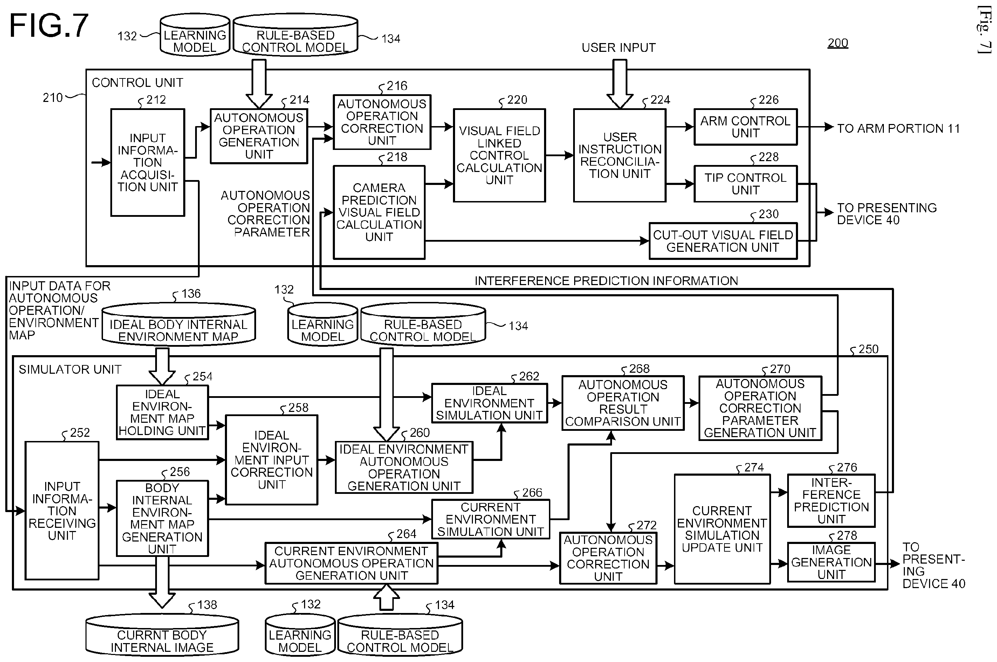

<4.3 Example of detailed configuration of autonomous operation execution unit 200> Next, the stage in which the autonomous operation is performed (autonomous operation execution stage) according to the present embodiment will be described. An example of a detailed configuration of the autonomous operation execution unit 200 according to the embodiment of the present disclosure will be described with reference to FIG. 7. FIG. 7 is a block diagram illustrating an example of the configuration of the autonomous operation execution unit 200 according to the present embodiment. The autonomous operation execution unit 200 according to the present embodiment can control the robot arm device 10 (for example, the arm portion 11) to autonomously operate in real time based on various data (external input information). Specifically, as illustrated in FIG. 7, the autonomous operation execution unit 200 is mainly constituted by two units, a control unit 210 and a simulator unit 250. The autonomous operation execution unit 200 may be a device integrated with the robot arm device 10 or the control unit 20 illustrated in FIG. 4, may be a separate device, or may be a device that is provided on the cloud and is communicably connected to the robot arm device 10 and the control unit 20.

### - Control unit 210 -

First, the control unit 210 will be described. Specifically, as illustrated in FIG. 7, the control unit 210 includes an input information acquisition unit 212, an autonomous operation generation unit (control information generation unit) 214, an autonomous operation correction unit (correction unit) 216, a camera prediction visual field calculation unit 218, a visual field linked control calculation unit (avoidance control information generation unit) 220, a user instruction reconciliation unit 224, an arm control unit (control unit) 226, a tip control unit 228, and a cut-out visual field generation unit (prediction image generation unit) 230. Details of each functional unit of the control unit 210 will be sequentially described below.

### (Input information acquisition unit 212)

The input information acquisition unit 212 acquires various data (external input information) obtained in actual surgery or the like (actual environment) currently being performed and outputs the data to the autonomous operation generation unit 214 and the simulator unit 250, which will be described later. In the present embodiment, examples of the various data include tip position/posture data of forceps held in both left and right hands (position information and posture information of a medical instrument in an actual environment), tip position/posture data of the imaging unit 12 (position information and posture information of a medical arm in an actual environment), an image taken by the imaging unit 12 (an image of an actual environment taken by a medical observation device), and sensing data from a distance measuring sensor (e.g., a stereo endoscope, a depth sensor). In the present embodiment, the tip position/posture data may be acquired in real time by using sensing data from a motion sensor provided on the arm portion 11 or the distance measuring sensor described above. Further, in the present embodiment, when a master slave method is used for an operation, the tip position/posture data may be generated by using information input to the input device 5047 (master console). In the present embodiment, information on the tip position/posture data may be expressed as an absolute coordinate value or may be expressed as a relative coordinate value from a predetermined reference point, and is not limited in particular.

### (Autonomous operation generation unit 214)

The autonomous operation generation unit 214 can generate an autonomous operation target value (autonomous operation control information) for allowing the robot arm device 10 (for example, the arm portion 11) (medical arm) to autonomously operate based on various data (external input information) output from the input information acquisition unit 212. Specifically, the autonomous operation generation unit 214 generates, as the autonomous operation target value, information on position/ posture of the imaging unit 12 (e.g., position/posture of the imaging unit 12 for capturing only forceps within a field of view) based on actual tip position/posture data of forceps held in both left and right hands by referring to the learning model 132 and the rule-based control model 134. Alternatively, the autonomous operation generation unit 214 generates, as the autonomous operation target value, visual field information including information such as a position of a gazing point located at a center of a field of view of the imaging unit 12, a distance from the imaging unit 12 to the gazing point, and a visual field direction to the gazing point. Then, the autonomous operation generation unit 214 outputs the generated autonomous operation target value to the autonomous operation correction unit 216, which will be described later.

### (Autonomous operation correction unit 216)

The autonomous operation correction unit 216 can correct the autonomous operation target value (autonomous operation control information) in real time based on a result of operation simulation of the robot arm device 10 (for example, the arm portion 11). Specifically, the autonomous operation correction unit 216 can correct the autonomous operation target value (autonomous operation control information) in real time based on a result of operation simulation of the robot arm device 10 (for example, the arm portion 11) based on an actual current body internal environment (actual environment) and a result of operation simulation of the robot arm device 10 (for example, the arm portion 11) based on an ideal body internal environment (ideal or reference environment). More specifically, the autonomous operation correction unit 216 corrects the autonomous operation target value generated by the autonomous operation generation unit 214 in real time based on an autonomous operation correction parameter (difference) acquired from the simulator unit 250, which will be described later. Then, the autonomous operation correction unit 216 outputs the corrected autonomous operation target value to the visual field linked control calculation unit 220, which will be described later.

### (Camera prediction visual field calculation unit 218)

The camera prediction visual field calculation unit 218 decides a target field of view of the imaging unit 12 for prior presentation and collision avoidance of an interference target object based on interference prediction information (e.g., a position of an organ predicted to interfere, position/posture information of the imaging unit 12 during interference) acquired from the simulator unit 250, which will be described later. Specifically, in a case where the imaging unit 12 is an endoscope with a tip bending function, the camera prediction visual field calculation unit 218 outputs information on a target posture of a tip and a target field of view of the imaging unit 12 to the visual field linked control calculation unit 220, which will be described later. In a case where the imaging unit 12 is an endoscope with a wide angle/cutting out function, the camera prediction visual field calculation unit 218 outputs information on a range of a field of view to be cut out or a field of view to the cut-out visual field generation unit 230, which will be described later.

### (Visual field linked control calculation unit 220)

The visual field linked control calculation unit 220 can generate control information (tip freedom control command value, arm control command value) for allowing the robot arm device 10 (for example, the arm portion 11) to operate while avoiding interference based on the corrected autonomous operation target value and the target posture (interference prediction information) of the tip of the imaging unit 12. That is, in the present embodiment, in a case where the imaging unit 12 is an endoscope with a tip bending function, the visual field linked control calculation unit 220 can generate control information for controlling the arm portion 11 and the tip of the imaging unit 12 to move in association with each other. Then, the visual field linked control calculation unit 220 outputs the generated control information to the user instruction reconciliation unit 224, which will be described later. In a case where the imaging unit 12 is not an endoscope with a tip bending function, the visual field linked control calculation unit 220 outputs the corrected autonomous operation target value to the user instruction reconciliation unit 224 as it is.

### (User instruction reconciliation unit 224)

The user instruction reconciliation unit 224 can update control information (tip control command value, arm control command value) based on the control information (control command value) output from the visual field linked control calculation unit 220 and a correction instruction (for example, gazing point offset, zoom amount offset) manually input by the surgeon (user) 5067. Then, the user instruction reconciliation unit 224 outputs the updated control information (tip control command value, arm control command value) to the arm control unit 226 and the tip control unit 228, which will be described later. In the present embodiment, in a case where there is no input from the surgeon (user) 5067, the control information (control command value) output from the visual field linked control calculation unit 220 is output to the arm control unit 226 and the tip control unit 228 as it is.

### (Arm control unit 226)

The arm control unit 226 can control the arm portion (medical arm) 11 based on the updated control information (the arm control command value).

### (Tip control unit 228)

The tip control unit 228 can control a direction (visual field direction) of the tip of the imaging unit 12, a zoom amount, a posture of the tip of the imaging unit 12, and the like in synchronization with the arm control unit 226 based on the updated control information (tip control command value).

### (Cut-out visual field generation unit 230)

The cut-out visual field generation unit 230 generates a prediction image that is predicted to be cut out in a case where cutting-out processing is performed on a wide-angle image acquired by the imaging unit 12 based on a range of a field of view to be cut out and the field of view obtained from the camera prediction visual field calculation unit 218 and outputs the prediction image to the presenting device 40.

### - Simulator unit 250 -

Next, the simulator unit 250 will be described. The simulator unit 250 can perform an operation simulation of the robot arm device 10 (for example, the arm portion 11) based on various data (external input information). Specifically, as illustrated in FIG. 7, the simulator unit 250 mainly includes an input information receiving unit 252, an ideal environment map holding unit (reference environment map holding unit) 254, a body internal environment map generation unit 256, an ideal environment input correction unit (reference environment input correction unit)258, an ideal environment autonomous operation generation unit 260(reference environment autonomous operation generation unit), an ideal environment simulation unit (reference environment simulation unit)262, a current environment autonomous operation generation unit 264, a current environment simulation unit (actual environment simulation unit) 266, an autonomous operation result comparison unit (comparison unit) 268, an autonomous operation correction parameter generation unit 270, an autonomous operation correction unit 272, a current environment simulation update unit (re-simulation unit) 274, an interference prediction unit (prediction unit) 276, and an image generation unit 278. Details of each functional unit of the simulator unit 250 will be sequentially described below.

### (Input information receiving unit 252)

The input information receiving unit 252 outputs data used for environment map generation to the body internal environment map generation unit 256 from the input information acquisition unit 212 of the control unit 210 described above, and outputs data used for generation of data for autonomous operation to the ideal environment input correction unit 258 and the current environment autonomous operation generation unit 264. Further, the input information receiving unit 252 outputs, to the body internal environment map generation unit 256, tip position/posture data of forceps held in both left and right hands, tip position/posture data of the imaging unit 12, an image taken by the imaging unit 12, and sensing data from a distance measuring sensor.

### (Ideal environment map holding unit 254)

The ideal environment map holding unit 254 stores therein the ideal body internal environment map 135 generated by the ideal map generation unit 124, and outputs the ideal body internal environment map 135 to the ideal environment input correction unit 258 and the ideal environment simulation unit 262.

### (Body internal environment map generation unit 256)

The body internal environment map generation unit 256 can generate an actual current body internal environment map (actual environment map) in real time based on various data (external input information) including tip position/posture data of forceps held in both left and right hands, tip position/posture data of the imaging unit 12, an image taken by the imaging unit 12, and sensing data from a distance measuring sensor that are acquired from the input information receiving unit 252. For example, the body internal environment map generation unit 256 generates a current body internal environment map based on an image taken by the imaging unit 12 by using SLAM. The generated current body internal environment map is used for data correction based on comparison with an ideal body internal environment map by the ideal environment input correction unit 258, which will be described later. Three-dimensional map information (body internal environment map) in a body currently undergoing surgery (referred to as a current environment (actual environment) or a current body internal environment) is referred to as a "current body internal environment map".

### (Ideal environment input correction unit 258)

The ideal environment input correction unit 258 extracts feature points of the ideal body internal environment map information stored in the ideal environment map holding unit 254 and feature points of the current body internal environment map generated by the body internal environment map generation unit 256, for example, by using current tip position/posture data of the imaging unit 12, tip position/posture data of forceps held in both left and right hands, and the like and calculates a difference between the extracted feature points. Then, the ideal environment input correction unit 258 can correct the data (external input information) used for generation of data for autonomous operation acquired from the input information receiving unit 252 based on the calculated difference. For example, the ideal environment input correction unit 258 outputs the corrected various data (external input information) such as tip position/ posture data of forceps held by both left and right hands in an ideal environment to the ideal environment autonomous operation generation unit 260.

### (Ideal environment autonomous operation generation unit 260)

The ideal environment autonomous operation generation unit 260 can generate an autonomous operation target value (autonomous operation control information in the ideal or reference environment) of the robot arm device 10 (for example, the arm portion 11) based on the corrected various data (external input information) by referring to the learning model 132 or the rule-based control model 134. Specifically, the ideal environment autonomous operation generation unit 260 generates, as the autonomous operation target value, information on position/posture of the imaging unit 12 (for example, position/posture of the imaging unit 12 for capturing only forceps within a field of view) based on tip position/posture data of forceps held in both left and right hands by referring to the learning model 132 and the rule-based control model 134 described above. Alternatively, the ideal environment autonomous operation generation unit 260 generates, as the autonomous operation target value, visual field information including information on a position of a gazing point located at a center of the field of view of the imaging unit 12, a distance from the imaging unit 12 to the gazing point, and a visual field direction to the gazing point. Then, the ideal environment autonomous operation generation unit 260 outputs the generated autonomous operation target value to the ideal environment simulation unit 262.

### (Ideal environment simulation unit 262)

The ideal environment simulation unit 262 can perform an operation simulation of the robot arm device 10 (for example, the arm portion 11) in an ideal or reference environment based on the ideal body internal environment map (ideal or reference environment map) stored in the ideal environment map holding unit 254 and the autonomous operation target value generated by the ideal environment autonomous operation generation unit 260. The ideal environment simulation unit 262 outputs, as a simulation result, an image taken by the imaging unit 12 in the ideal body internal environment, information on a positional relationship between the imaging unit 12 and forceps, and information on a positional relationship between the imaging unit 12 and peripheral organs to the autonomous operation result comparison unit 268.

### (Current environment autonomous operation generation unit 264)

The current environment autonomous operation generation unit 264 can generate an autonomous operation target value (autonomous operation control information in an actual environment) of the robot arm device 10 (for example, the arm portion 11) based on various data (external input information) from the input information receiving unit 252 by referring to the learning model 132 and the rule-based control model 134. Specifically, the current environment autonomous operation generation unit 264 generates, as the autonomous operation target value, information on position/posture of the imaging unit 12 (for example, position/posture of the imaging unit 12 for capturing only forceps within a field of view) based on tip position/posture data of forceps held in both left and right hands by referring to the learning model 132 and the rule-based control model 134 described above. Alternatively, the current environment autonomous operation generation unit 264 generates, as the autonomous operation target value, visual field information including information such as a position of a gazing point located at a center of a field of view of the imaging unit 12, a distance from the imaging unit 12 to the gazing point, and a visual field direction to the gazing point. Then, the current environment autonomous operation generation unit 264 outputs the generated autonomous operation target value to the current environment simulation unit 266, which will be described later.

### (Current environment simulation unit 266)

The current environment simulation unit 266 can perform an operation simulation of the robot arm device 10 (for example, the arm portion 11) in a current environment (actual environment) based on the current body internal environment map (actual environment map) generated by the body internal environment map generation unit 256 in real time and the autonomous operation target value generated by the current environment autonomous operation generation unit 264. The current environment simulation unit 266 outputs, as a simulation result, an image taken by the imaging unit 12 in a current body internal environment, information on a positional relationship between the imaging unit 12 and forceps, and information on a positional relationship between the imaging unit 12 and peripheral organs to the autonomous operation result comparison unit 268.

### (Autonomous operation result comparison unit 268)

The autonomous operation result comparison unit 268 can extract a difference between the result of the operation simulation in the ideal environment performed by the ideal environment simulation unit 262 and the result of the operation simulation in the current environment (actual environment) performed by the current environment simulation unit 266. Specifically, the autonomous operation result comparison unit 268 outputs, as the difference, a difference in feature points of the image taken by the imaging unit 12, a difference in positional relationship between the imaging unit 12 and the forceps, and a difference in positional relationship between the imaging unit 12 and the peripheral organs to the autonomous operation correction parameter generation unit 270 based on the image taken by the imaging unit 12 in the ideal environment, the image taken by the imaging unit 12 in the current environment, the information on the positional relationships between the imaging unit and the forceps in the ideal environment and the current environment, the information on the positional relationships between the imaging unit 12 and the peripheral organs, and the like.

### (Autonomous operation correction parameter generation unit 270)

The autonomous operation correction parameter generation unit 270 generates a correction parameter (offset and a zoom amount of position/posture of the imaging unit 12) for correcting the autonomous operation target of the robot arm device 10 (for example, the arm portion 11) based on the difference acquired from the autonomous operation result comparison unit 268. Then, the autonomous operation correction parameter generation unit 270 outputs the generated correction parameter to the autonomous operation correction units 216 and 272.

### (Autonomous operation correction unit 272)

The autonomous operation correction unit 272 can correct the autonomous operation target value (autonomous operation control information in the actual environment) of the robot arm device 10 (for example, the arm portion 11) based on the autonomous operation target value (autonomous operation control information in the actual environment) of the robot arm device 10 (for example, the arm portion 11) generated by the current environment autonomous operation generation unit 264 and the correction parameter (offset and a zoom amount of position/posture of the imaging unit 12) generated by the autonomous operation correction parameter generation unit 270. Then, the autonomous operation correction unit 272 outputs the corrected autonomous operation target value to the current environment simulation update unit 274, which will be described later.

### (Current environment simulation update unit 274)

The current environment simulation update unit 274 can perform an operation simulation in the current environment again based on the autonomous operation target value corrected by the autonomous operation correction unit 272. Specifically, the current environment simulation update unit 274 can perform an operation simulation of the robot arm device 10 (for example, the arm portion 11) in the current environment again based on the current body internal environment map (actual environment map) updated in real time by the body internal environment map generation unit 256 and the autonomous operation target value corrected by the autonomous operation correction unit 272. Then, the current environment simulation update unit 274 outputs, as a simulation result, the image taken by the imaging unit 12, information on the positional relationship between the imaging unit 12 and the forceps, and information on the positional relationship between the imaging unit 12 and the peripheral organs in the current environment to the interference prediction unit 276 and the image generation unit 278, which will be described later.

### (Interference prediction unit 276)

The interference prediction unit 276 can perform interference prediction, contact judgment, and the like with the peripheral organs (other objects) based on the result of the re-simulation performed by the current environment simulation update unit 274 and can output interfere prediction information (information on positions of interference organs (other portions) predicted to interfere, position/posture of the imaging unit 12 (or the arm portion 11) during interference, and the like) to the control unit 210.

### (Image generation unit 278)

The image generation unit 278 can generate a prediction image (interference prediction image) that is predicted to be obtained by the imaging unit 12 during interference with the interfering organs based on the result of the re-simulation performed by the current environment simulation update unit 274 and output the prediction image to the presenting device 40.

In the present embodiment, the detailed configuration of the autonomous operation execution unit 200 is not limited to the configuration illustrated in FIG. 7.

### - Presenting device 40 -

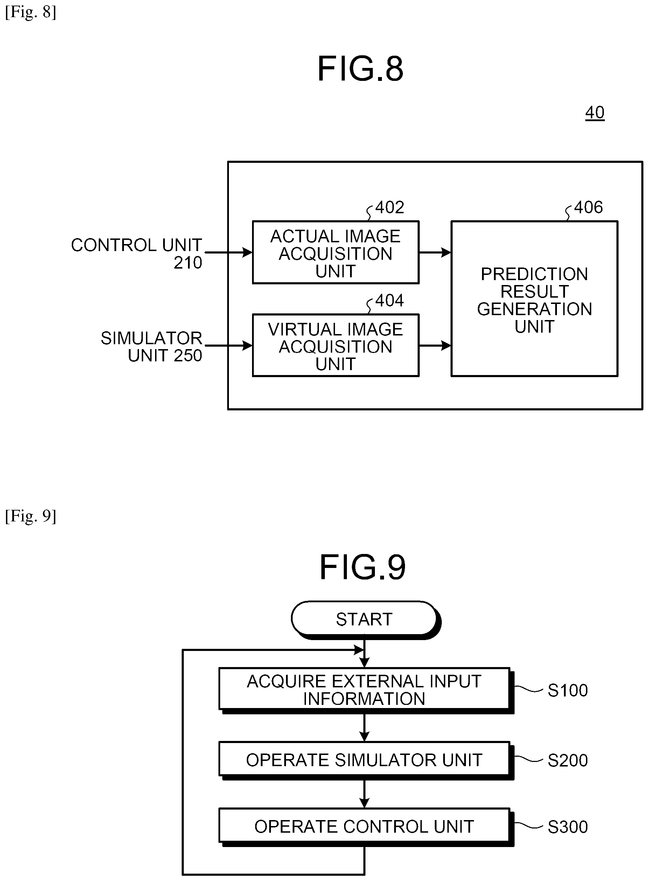

Next, the presenting device 40 will be described with reference to FIG. 8. FIG. 8 is a block diagram illustrating an example of the configuration of the presenting device 40 according to the present embodiment. Specifically, as illustrated in FIG. 8, the presenting device 40 mainly includes an actual image acquisition unit 402, a virtual image acquisition unit 404, and a prediction result generation unit 406. Details of each functional unit of the presenting device 40 will be sequentially described below.

### (Actual image acquisition unit 402)

The actual image acquisition unit 402 can acquire, from the control unit 210, an actual image (for example, a wide-angle image) from the imaging unit 12 and a prediction image that is predicted to be cut out generated by the cut-out visual field generation unit 230 described above and output the actual image and the prediction image to the prediction result generation unit 406, which will be described later.

### (Virtual image acquisition unit 404)

The virtual image acquisition unit 404 can acquire an interference prediction image that is predicted to be obtained by the imaging unit 12 during interference with an interfering organ from the simulator unit 250 and output the interference prediction image to the prediction result generation unit 406, which will be described later.

### (Prediction result generation unit 406)

The prediction result generation unit 406 can concurrently present, to the surgeon (user) 5067, any one, two, or three of an actual image (for example, a wide-angle image) from the imaging unit 12, a prediction image predicted to be cut out, and a prediction image predicted from the simulator unit 250 (for example, an interference prediction image predicted to be obtained by the imaging unit 12 during interference with an interfering organ).

In the present embodiment, the detailed configuration of the presenting device 40 is not limited to the configuration illustrated in FIG. 8.

### <4.4 Control method in autonomous operation execution stage>

### - Overview -

Next, a control method in the autonomous operation execution stage according to the present embodiment will be described. First, an outline of the control method according to the present embodiment will be described with reference to FIG. 9. FIG. 9 is a flowchart of the control method in the autonomous operation execution stage according to the present embodiment.

As illustrated in FIG. 9, the control method in the autonomous operation execution stage according to the present embodiment can mainly include steps from step S100 to step S300. The outline of each of these steps will be described below. The control method described below is started by acquisition of various data (external input information), and is repeatedly executed until a surgical task of the autonomous operation execution unit 200 is completed.

First, the autonomous operation execution unit 200 acquires various data (external input information) such as tip position/posture data of forceps held in both left and right hands (position information and posture information of a medical instrument in an actual environment), tip position/posture data of the imaging unit 12 (position information and posture information of a medical arm in the actual environment), an image taken by the imaging unit 12 (image taken in the actual environment by a medical observation device), and sensing data from a distance measuring sensor (e.g., a stereo endoscope, a depth sensor) (step S100).

Next, the autonomous operation execution unit 200 mainly operates the simulator unit 250 based on the acquired various data to perform a simulation (step S200).

Further, the autonomous operation execution unit 200 mainly operates the control unit 210 to control the arm portion 11 based on a result of the simulation in step S200 (step S300). The autonomous operation execution unit 200 repeatedly executes the flow illustrated in FIG. 9 until a task is completed.

### - Simulator unit operation stage -

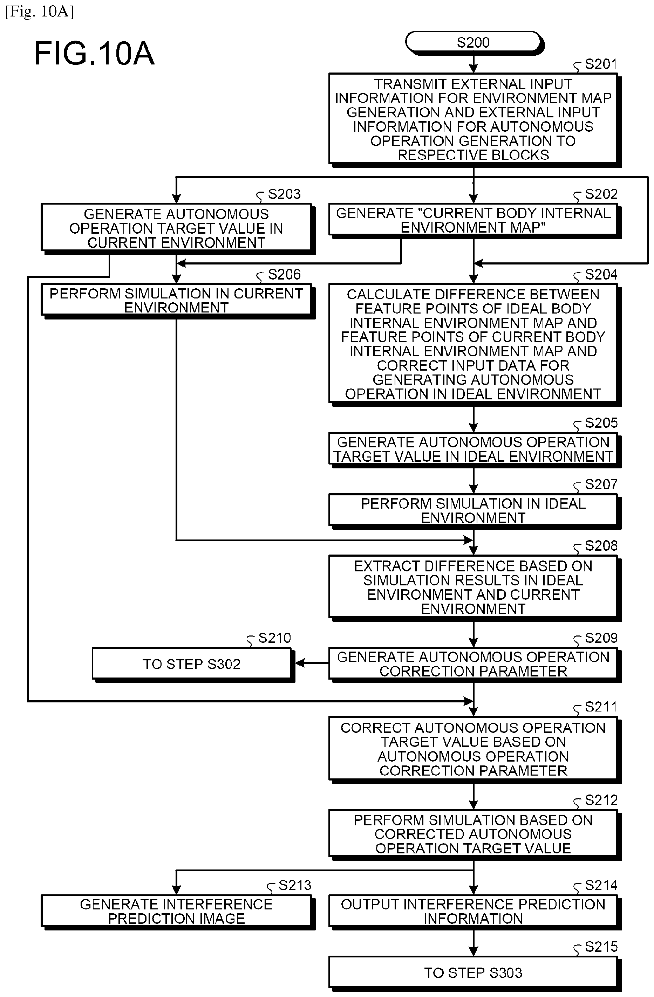

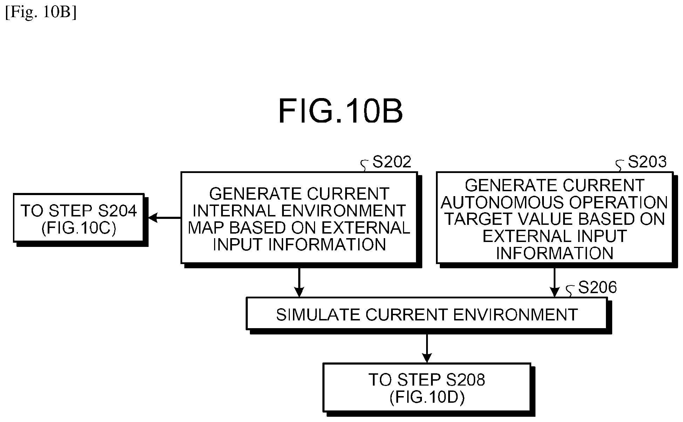

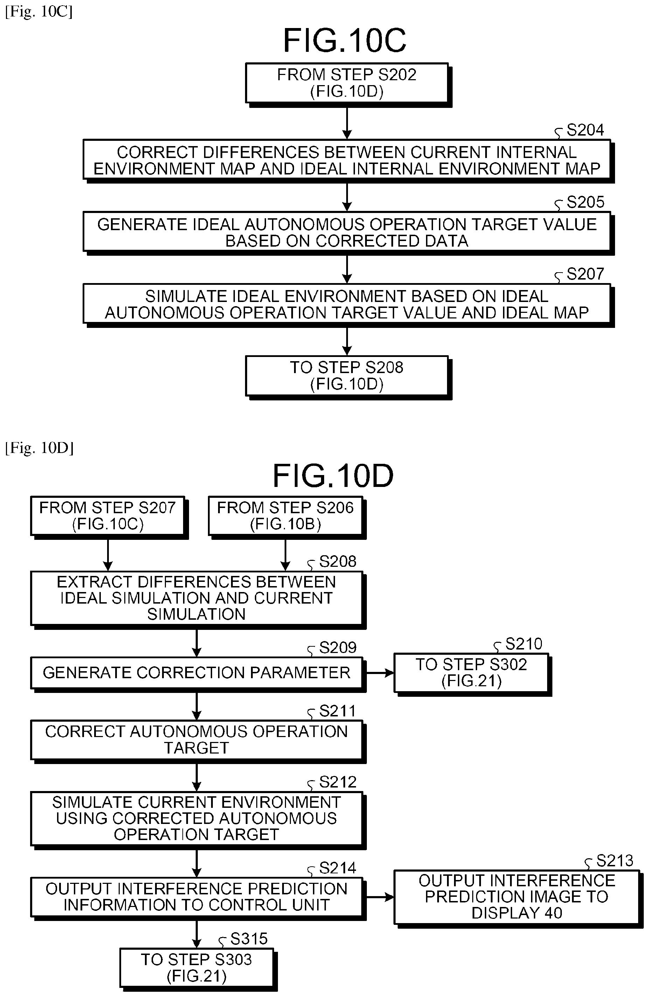

Next, details of step S200 illustrated in FIG. 9 will be described with reference to FIGS. 10 to 20. FIG. 10A is a sub-flowchart of step S200 illustrated in FIG. 9, and FIGS. 11 to 20 are explanatory views for explaining details of the control method according to the present embodiment. FIGS. 10B to 10D are sub-flow charts of step S200 showing the parallel steps of FIG. 10A for addition clarity. In particular, FIG. 10B is a sub-flowchart of steps to simulate the current environment; FIG. 10 C is a sub-flowchart of steps to simulate the ideal or reference environment; and FIG. 10D is a sub-flowchart of steps to simulate the current environment using corrected autonomous operation target.

As illustrated in FIG. 10A, step S200 can mainly include a plurality of substeps from substep S201 to substep S215. Details of each of these substeps will be described below.

The input information receiving unit 252 outputs data used for generation of an environment map to the body internal environment map generation unit 256 and outputs data used for autonomous operation to the ideal environment input correction unit 258 and the current environment autonomous operation generation unit 264 from the input information acquisition unit 212 of the control unit 210 described above. Further, the input information receiving unit 252 outputs, for example, tip position/posture data of forceps held in both left and right hands, tip position/posture data of the endoscope 5001, an image taken by the imaging unit 12, sensing data from a distance measuring sensor, and the like to the body internal environment map generation unit 256 (substep S201).

The body internal environment map generation unit 256 generates a current body internal environment map (actual environment map) in real time based on various data (external input information) including the tip position/posture data of the forceps held in both left and right hands, the tip position/posture data of the imaging unit 12, the image taken by the imaging unit 12, and the sensing data from the distance measuring sensor) that are acquired from the input information receiving unit 252 (substep S202).

The autonomous operation generation unit 214 generates an autonomous operation target value (autonomous operation control information) for causing the robot arm device 10 (for example, the arm portion 11) to autonomously operate based on the various data (external input information) output from the input information acquisition unit 212 (substep S203).

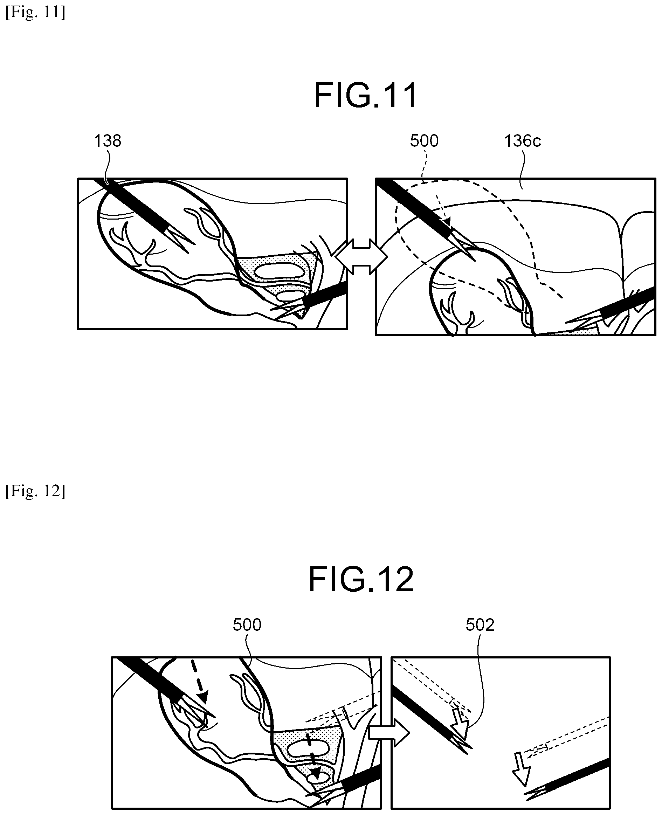

The ideal environment input correction unit 258 extracts feature points of the ideal body internal environment map information stored in the ideal environment map holding unit 254 and feature points of the current body internal environment map generated by the body internal environment map generation unit 256, for example, by using current tip position/posture data of the imaging unit 12, tip position/posture data of forceps ) held in both left and right hands, and the like and calculates a difference between the extracted feature points. For example, as illustrated in FIG. 11, the ideal environment input correction unit 258 can extract a difference of 500 from an image 136c in the ideal environment by extracting feature points of a contour of an organ in a current body internal image 138.

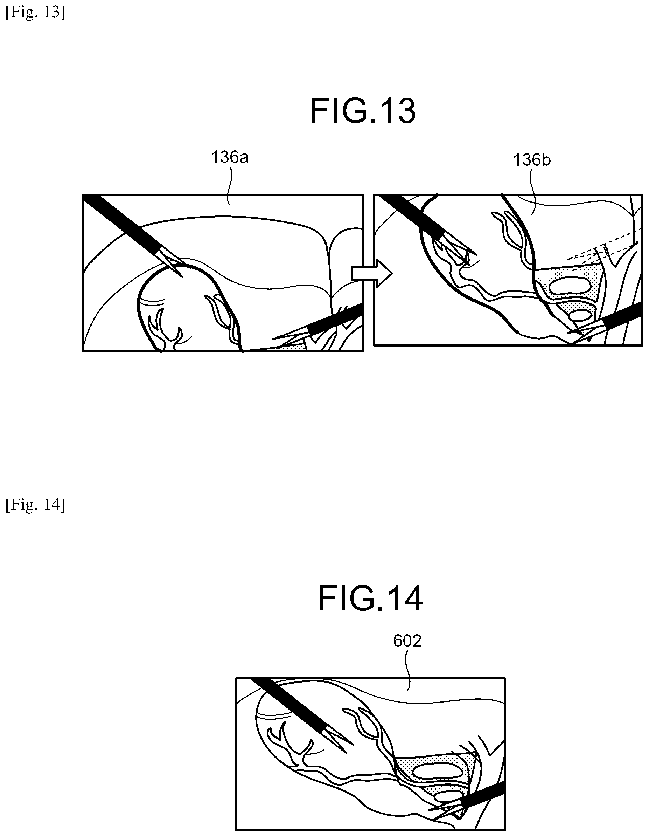

Then, the ideal environment input correction unit 258 corrects input data used for autonomous operation in the ideal environment acquired from the input information receiving unit 252 based on the difference. For example, as illustrated in FIG. 12, the ideal environment input correction unit 258 can calculate a correction amount 502 for correcting positions of the forceps based on the difference 500 in position of the organ. Then, the ideal environment input correction unit 258 can correct the positions of the forceps, for example, from the state of an image 136a illustrated on the left side of FIG. 13 to the state of an image 136b illustrated on the right side of FIG. 13 based on the calculated correction amount (substep S204).

The ideal environment autonomous operation generation unit 260 generates an autonomous operation target value (autonomous operation control information in the ideal environment) of the robot arm device 10 (for example, the arm portion 11) in the ideal or reference environment based on the corrected various data (external input information) by referring to the learning model 132 and the rule-based control model 134 (substep S205).

The current environment autonomous operation generation unit 264 generates an autonomous operation target value (autonomous operation control information in the actual environment) of the robot arm device 10 (for example, the arm portion 11) in the current environment based on the various data (external input information) from the input information receiving unit 252 by referring to the learning model 132 and the rule-based control model 134. Then, the current environment simulation unit 266 performs an operation simulation of the robot arm device 10 (for example, the arm portion 11) in the current environment based on the current body internal environment map (actual environment map) updated in real time by the body internal environment map generation unit 256 and the autonomous operation target value generated by the current environment autonomous operation generation unit 264 (substep S206). For example, the current environment simulation unit 266 performs a simulation of an image 602 obtained by the imaging unit 12 in the current environment, as illustrated in FIG. 14.

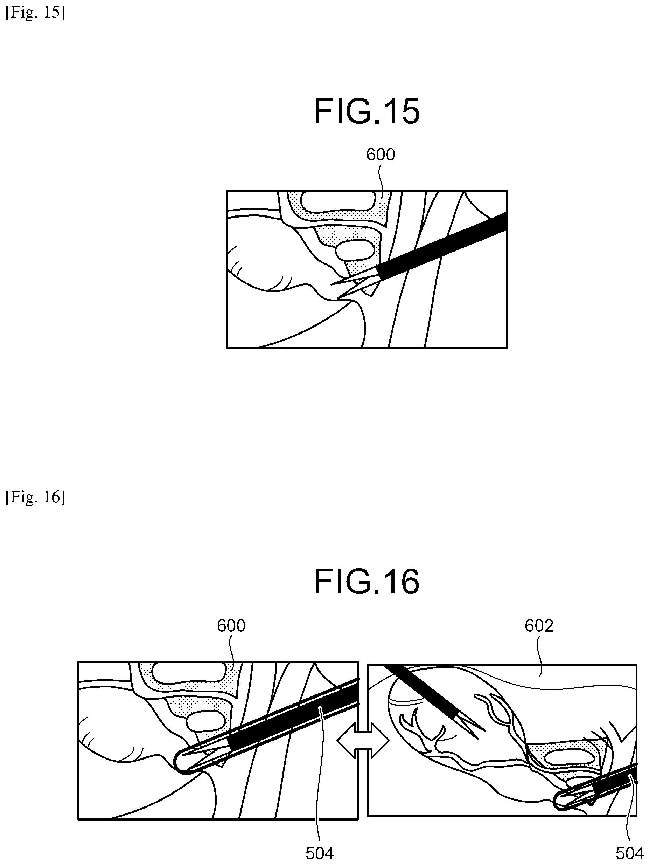

The ideal environment simulation unit 262 performs an operation simulation of the robot arm device 10 (for example, the arm portion 11) in the ideal environment based on the ideal body internal environment map information stored in the ideal environment map holding unit 254 and the autonomous operation target value generated by the ideal environment autonomous operation generation unit 260 (substep S207). For example, the ideal environment simulation unit 262 performs a simulation of an image 600 obtained by the imaging unit 12 in the ideal environment (substep S207), as illustrated in FIG. 15.

The autonomous operation result comparison unit 268 extracts a difference between a result of the operation simulation in the ideal environment performed by the ideal environment simulation unit 262 and a result of the operation simulation in the current environment performed by the current environment simulation unit 266 (substep S208). For example, as illustrated in FIG. 16, the autonomous operation result comparison unit 268 extracts a difference 504 between the image 600 taken by the imaging unit 12, which is the result of the operation simulation in the ideal environment performed by the ideal environment simulation unit 262, and the image 602 taken by the imaging unit 12, which is the result of the operation simulation in the current environment performed by the current environment simulation unit 266.

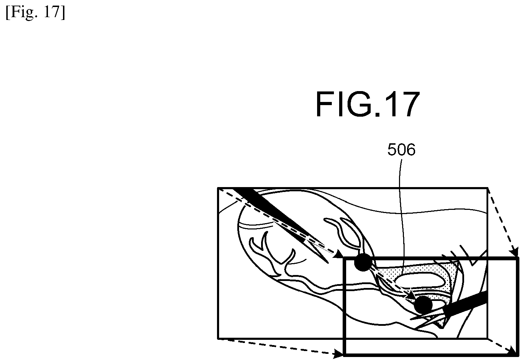

The autonomous operation correction parameter generation unit 270 generates a correction parameter (offset and zoom amounts of the position/posture of the imaging unit 12) for correcting the autonomous operation target of the robot arm device 10 (for example, the arm portion 11) based on the difference acquired from the autonomous operation result comparison unit 268 (substep S209). For example, the autonomous operation correction parameter generation unit 270 calculates an offset amount and a zoom amount of the gazing point of the imaging unit 12 as a correction parameter 506 as illustrated in FIG. 17.

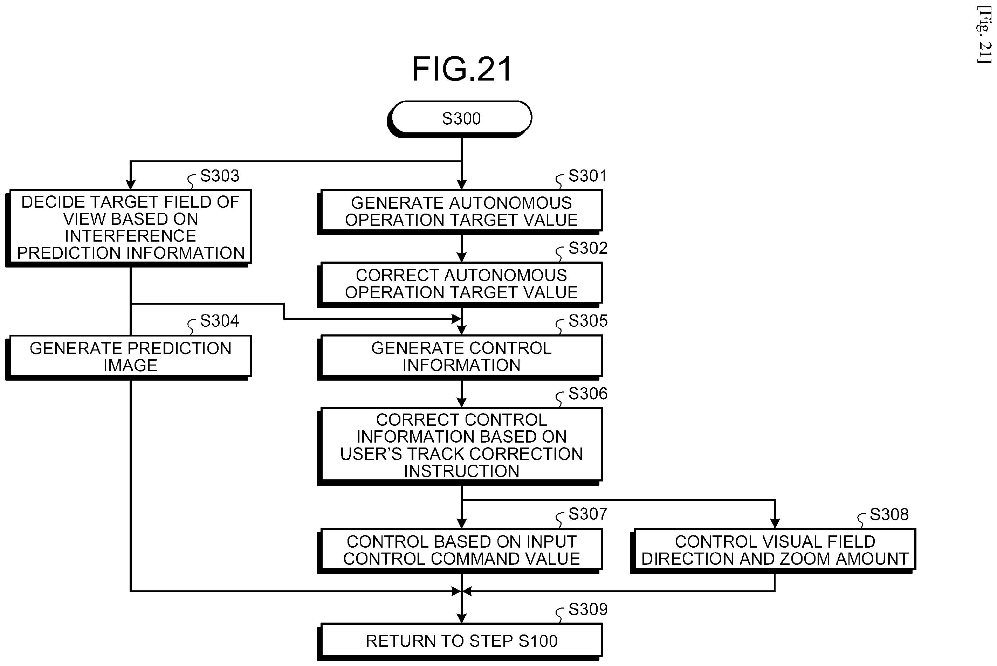

The autonomous operation execution unit 200 proceeds to the process in substep S302 illustrated in FIG. 21 (substep S210).

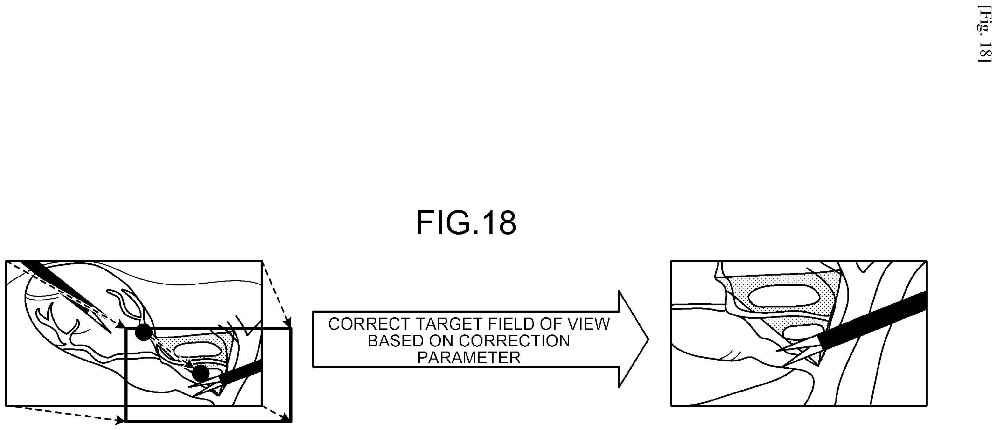

The autonomous operation correction unit 272 corrects the autonomous operation target value of the robot arm device 10 (for example, the arm portion 11) based on the autonomous operation target value (autonomous operation control information in the actual environment) of the robot arm device 10 (for example, the arm portion 11) generated by the current environment autonomous operation generation unit 264 and the correction parameter (offset and zoom amounts of the position/posture of the imaging unit 12) generated by the autonomous operation correction parameter generation unit 270 (substep S211). For example, the autonomous operation correction unit 272 corrects a target field of view of the imaging unit 12 based on the correction parameter 506, as illustrated in FIG. 18.

The current environment simulation update unit 274 performs an operation simulation in the current body internal environment again based on the autonomous operation target value corrected by the autonomous operation correction unit 272 (substep S212).

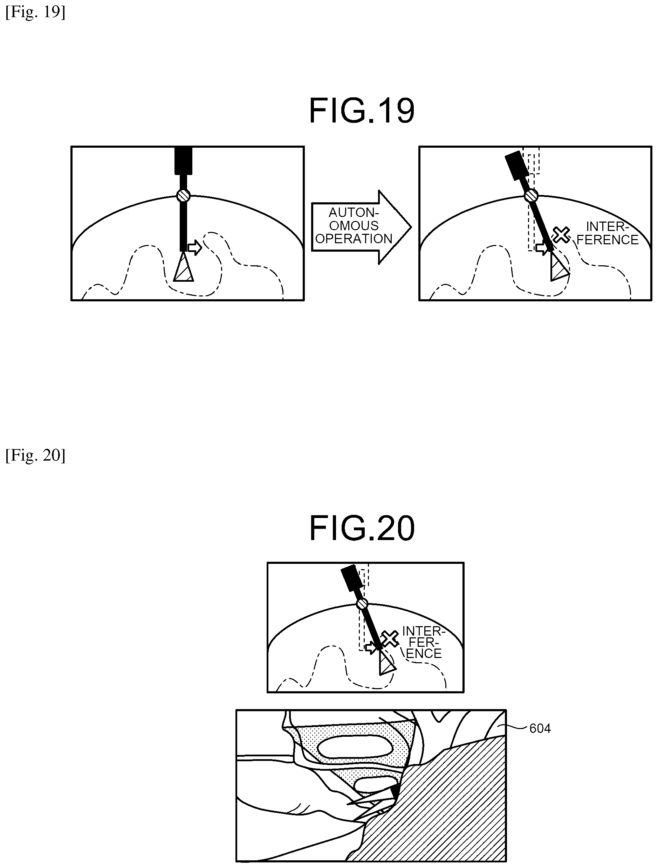

The interference prediction unit 276 performs interference prediction and contact determination with a surrounding organ based on the result of the re-simulation performed by the current environment simulation update unit 274 and outputs interference prediction information to the control unit 210 (substep S214). For example, the interference prediction unit 276 detects interference with a surrounding organ in a simulation, as illustrated in FIG. 19.

The image generation unit 278 generates an interference prediction image that is predicted to be obtained during predicted interference with a surrounding organ based on the result of the re-simulation performed by the current environment simulation update unit 274 and outputs the interference prediction image to the presenting device 40 (substep S213). For example, the image generation unit 278 generates an interference prediction image 604 as illustrated in FIG. 20.

The autonomous operation execution unit 200 proceeds to the process in substep S303 illustrated in FIG. 21 (substep S215).

### - Control unit operation stage -

Next, details of step S300 illustrated in FIG. 9 will be described with reference to FIGS. 21 to 25. FIG. 21 is a sub-flow chart of step S300 illustrated in FIG. 9, and FIGS. 22 to 25 are explanatory views for explaining details of the control method according to the present embodiment. As illustrated in FIG. 21, step S300 can mainly include a plurality of substeps from substep S301 to substep S309. Details of each of these substeps will be described below.

The autonomous operation generation unit 214 generates an autonomous operation target value (autonomous operation control information) for causing the robot arm device 10 (for example, the arm portion 11) to autonomously operate based on various data (external input information) output from the input information acquisition unit 212 (substep S301).

The autonomous operation correction unit 216 corrects the autonomous operation target value (autonomous operation control information) in real time based on a result of an operation simulation of the robot arm device 10 (for example, the arm portion 11) in an actual current body internal environment (actual environment) and a result of an operation simulation of the robot arm device 10 (for example, the arm portion 11) in an ideal body internal environment (substep S302).

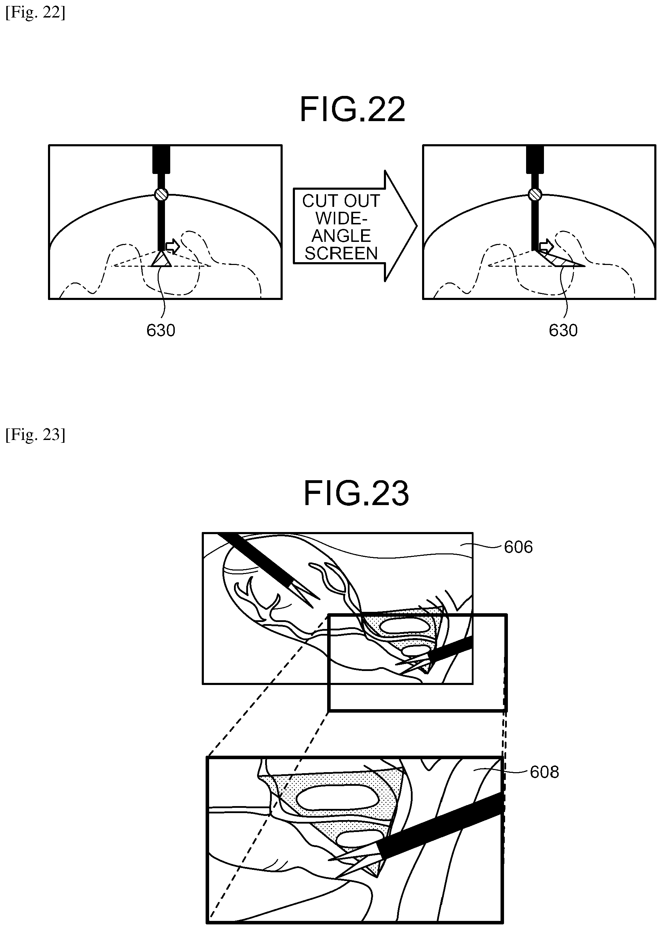

The camera prediction visual field calculation unit 218 decides a target field of view of the imaging unit 12 for prior presentation of interference and interference avoidance based on the interference prediction information acquired from the simulator unit 250 described later (substep S303). Specifically, for example, in a case where the imaging unit 12 is an endoscope with a wide angle/cutting out function, the camera prediction visual field calculation unit 218 changes a visual field range 630 to be cut out from a range illustrated on the left side of FIG. 22 to a range illustrated on the right side of FIG. 22.

In a case where the imaging unit 12 is an endoscope with a wide angle/cutting out function, the cut-out visual field generation unit 230 generates a prediction image 608 that is predicted to be cut out when cutting-out processing is performed on a wide-angle image 606 acquired by the imaging unit 12 as illustrated in FIG. 23 based on the visual field range to be cut out acquired from the camera prediction visual field calculation unit 218 (substep S304).

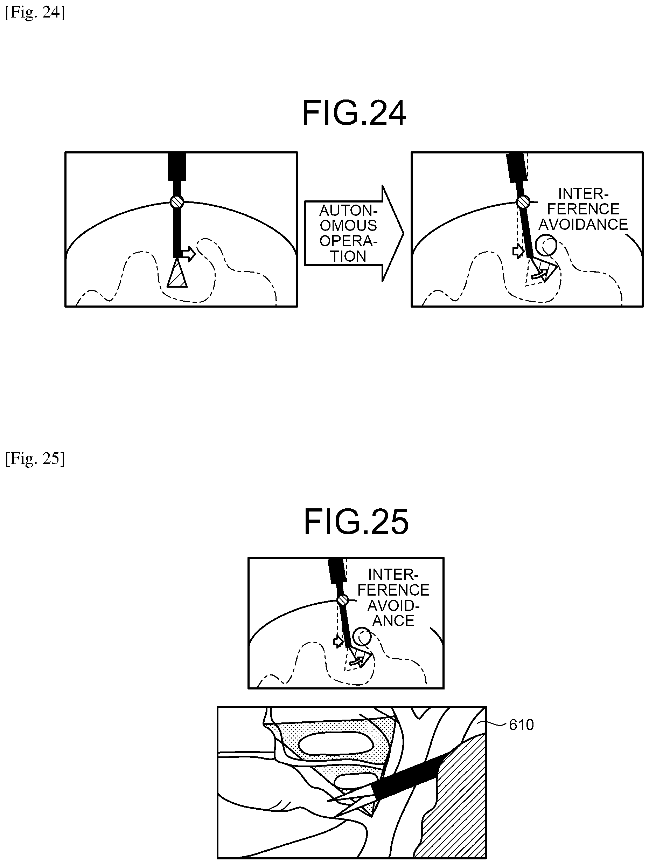

The visual field linked control calculation unit 220 generates control information (tip freedom control command value, arm control command value) for causing the robot arm device 10 (for example, the arm portion 11) to operate while avoiding interference based on the corrected autonomous operation target value and a target posture (the interference prediction information) of the tip of the imaging unit 12 (substep S305). For example, in a case where the imaging unit 12 is an endoscope with a tip bending function, the visual field linked control calculation unit 220 generates control information that allows the arm portion 11 and the tip of the imaging unit 12 to move in a linked manner while avoiding interference as illustrated in FIG. 24. In the present embodiment, a prediction image 612 that is predicted to be obtained by the imaging unit 12 in the case of avoidance of interference as illustrated in FIG. 25 may be generated based on the generated control information.

The user instruction reconciliation unit 224 corrects the control information (tip control command value, arm control command value) based on the control information (control command value) output from the visual field linked control calculation unit 220 and a correction instruction (for example, gazing point offset, zoom amount offset) manually entered by the surgeon (user) 5067 (substep S306).

The arm control unit 226 controls the arm portion 11 based on the updated control information (arm control command value) (substep S307).

The tip control unit 228 controls a visual field direction, a zoom amount, and the like of the imaging unit 12 based on the control information (tip control command value) (substep S308).

The autonomous operation execution unit 200 proceeds to the process in step S100 illustrated in FIG. 9 (substep S309).

### - Presentation stage -

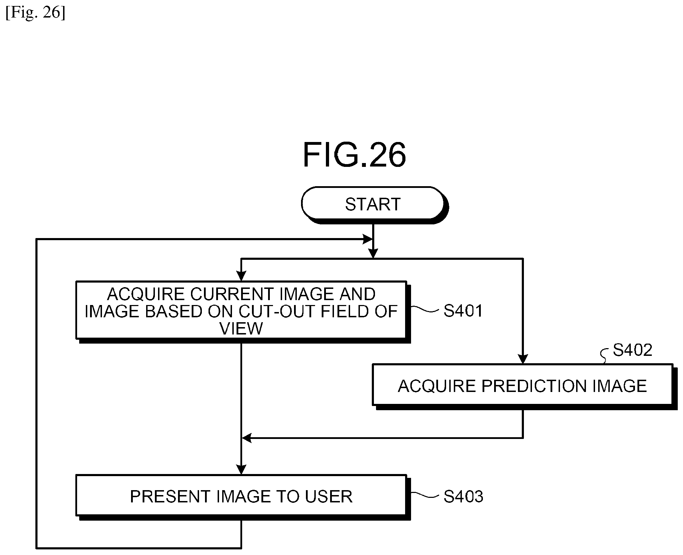

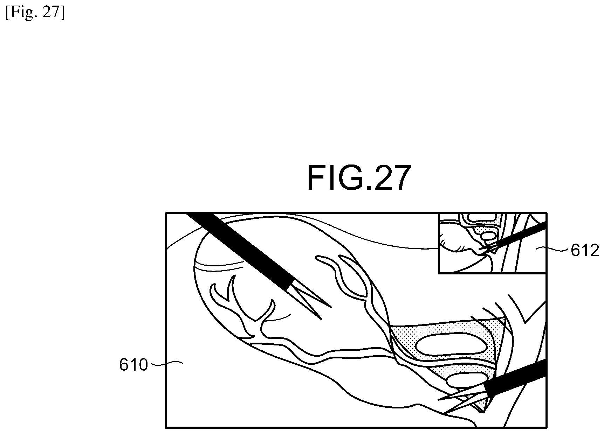

Next, a control method in the presentation stage according to the present embodiment will be described with reference to FIGS. 26 and 27. FIG. 26 is a flowchart of the control method according to the present embodiment, and FIG. 27 is an explanatory diagram for explaining details of the control method according to the present embodiment.

The presentation stage according to the present embodiment is executed following the autonomous operation execution stage. As illustrated in FIG. 26, the control method in the presentation stage can mainly include a plurality of substeps from step S401 to step S403. Details of each of these steps will be described below.

The actual image acquisition unit 402 acquires, from the control unit 210, an actual image (wide-angle image) (current image) 610 from the imaging unit 12 and a prediction image based on the cut-out field of view (step S401).

The virtual image acquisition unit 404 acquires an interference prediction image predicted from the simulator unit 250 (step S402).

As illustrated in FIG. 27, the prediction result generation unit 406 concurrently presents, to the surgeon (user) 5067, any one, two, or three of an actual image 610 from the imaging unit 12, the prediction image 612 based on the cut-out field of view to be cut out, and the interference prediction image predicted by the simulator unit 250 (step S403). In the present embodiment, the prediction image 612 based on the cut-out field of view to be cut out, the interference prediction image predicted by the simulator unit 250, and the like can be presented prior to an action of a doctor. This enables movement of a field of view of the doctor, a smooth forceps operation, and an action for avoiding interference or the like.

As described above, according to the embodiment of the present disclosure, autonomous operation control information suitable for various body internal environments can be generated in real time by correcting autonomous operation control information generated based on various data (external input information) based on a result of an operation simulation of the robot arm device 10 in an actual body internal environment and a result of an operation simulation of the robot arm device 10 in an ideal body internal environment. A doctor can perform surgery suitably and smoothly with assistance from the robot arm device 10 controlled in accordance with such autonomous operation control information.

### <4.5 Modifications>

The embodiment of the present disclosure can also be applied to an operation based on a master slave method. In this case, the imaging unit 12, forceps, and the like supported by the arm portion 11 can be remotely controlled by the surgeon (user) 5067 via the input device 5047 (master console) installed in a place away from an operating room or in the operating room.

### <<5. Hardware configuration>>

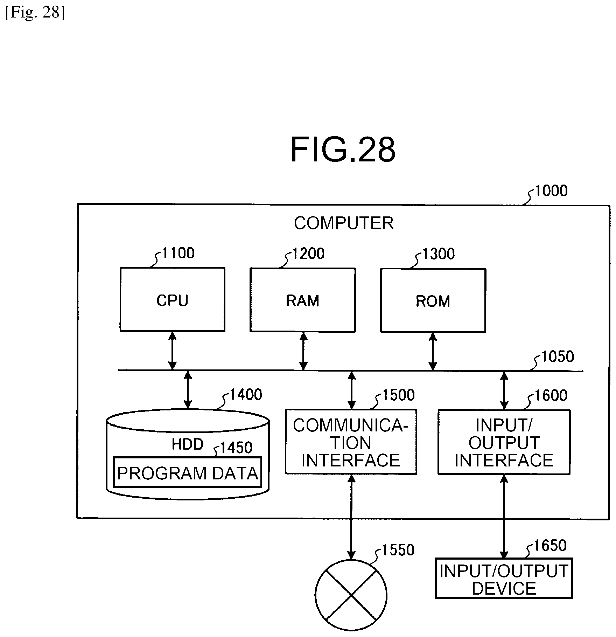

An information processing apparatus such as the autonomous operation execution unit 200 according to the embodiment described above is, for example, realized by a computer 1000 having a configuration illustrated in FIG. 28. The following discusses, as an example, the autonomous operation execution unit 200 according to the embodiment of the present disclosure. FIG. 28 is a hardware configuration diagram illustrating an example of the computer 1000 that realizes the functions of the autonomous operation execution unit 200 according to the embodiment of the present disclosure. The computer 1000 has a CPU 1100, a RAM 1200, a read only memory (ROM) 1300, a hard disk drive (HDD) 1400, a communication interface 1500, and an input/output interface 1600. The units of the computer 1000 are connected by a bus 1050.

The CPU 1100 operates based on a program stored in the ROM 1300 or the HDD 1400 and controls each unit. For example, the CPU 1100 loads programs stored in the ROM 1300 or the HDD 1400 into the RAM 1200 and executes processing corresponding to the various programs.

The ROM 1300 stores therein, for example, a boot program such as a basic input output system (BIOS) executed by the CPU 1100 when the computer 1000 is booted up and a program that depends on the hardware of the computer 1000.

The HDD 1400 is a computer-readable recording medium that records, for example, a program executed by the CPU 1100 and data used by the program in a non-transitory manner. Specifically, the HDD 1400 is a recording medium for recording a program for the medical arm control method according to the present disclosure, which is an example of program data 1450.

The communication interface 1500 is an interface for the computer 1000 to connect to an external network 1550 (for example, the Internet). For example, the CPU 1100 receives data from another device or transmits data generated by the CPU 1100 to another device via the communication interface 1500.

The input/output interface 1600 is an interface for connecting an input/output device 1650 and the computer 1000. For example, the CPU 1100 receives data from an input device such as a keyboard or a mouse via the input/output interface 1600. The CPU 1100 also transmits data to an output device such as a display, a speaker, or a printer via the input/output interface 1600. Further, the input/output interface 1600 may function as a media interface for reading a program or the like recorded on a predetermined computer-readable recording medium. The medium is, for example, an optical recording medium such as a digital versatile disc (DVD) or a phase change rewritable disk (PD), a magneto-optical recording medium such as a magneto-optical disk (MO), a tape medium, a magnetic recording medium, or a semiconductor memory.

For example, in a case where the computer 1000 functions as the autonomous operation execution unit 200 according to the embodiment of the present disclosure, the CPU 1100 of the computer 1000 realizes the functions of the simulator unit 250 and the like by executing a program for the medical arm control method loaded on the RAM 1200.

Further, the HDD 1400 may store therein a program for the medical arm control method according to the present disclosure and data in the storage unit 60. Although the CPU 1100 reads the program data 1450 from the HDD 1400 and executes the program data 1450, the CPU 1100 may, as another example, acquire an information processing program from another device over the external network 1550.

Further, the autonomous operation execution unit 200 according to the present embodiment may be applied to a system constituted by a plurality of devices, which is premised on connection to a network (or communication between devices), such as cloud computing. That is, the autonomous operation execution unit 200 according to the present embodiment described above can be realized as the medical observation system 1 according to the present embodiment, for example, by a plurality of devices.

An example of the hardware configuration of the autonomous operation execution unit 200 has been described above. Each of the above constituent elements may be a general-purpose member or may be hardware specialized for the function of the constituent element. Such a configuration can be appropriately changed depending on a technical level at the time of implementation.

### <<6. Supplement>>

The embodiment of the present disclosure described above can include, for example, an information processing method executed by an information processing apparatus or an information processing system as described above, a program for causing the information processing apparatus to function, and a non-transitory tangible medium on which the program is recorded. Further, the program may be distributed over a communication line (including wireless communication) such as the Internet.

Further, each step in the information processing method according to the embodiment of the present disclosure described above need not necessarily be performed in the order described above. For example, the order in which the steps are performed may be changed as appropriate. Further, some of the steps may be performed in parallel or individually instead of being performed in chronological order. Further, each step need not necessarily be performed according to the described method and may be performed, for example, by another method by another functional unit.

Among the processes described in the above embodiment, all or part of the processes described as being automatically performed can be performed manually or all or part of the processes described as being performed manually can be automatically performed by a known method. In addition, the processing procedure, specific names, and information including various data and parameters shown in the above document and drawings can be changed in any ways unless otherwise specified. For example, the various information illustrated in the drawings is not limited to the illustrated information.

Further, each constituent element of each of the illustrated devices is a functional concept and need not necessarily be physically configured as illustrated in the drawings. That is, a specific form of distribution/integration of each device is not limited to the one illustrated in the drawings, and all or part of each device may be functionally or physically distributed/integrated in any unit depending on various loads and usage conditions.

Although embodiments of the present disclosure has been described in detail above with reference to the accompanying drawings, the technical scope of the present disclosure is not limited to such an example. It is clear that a person skilled in the art of the present disclosure can arrive at various changes or modifications within the scope of the technical idea described in the claims, and it is understood that such changes or modifications also belongs to the technical scope of the present disclosure.

In addition, the effects described herein are merely explanatory or illustrative and are not restrictive. That is, the technique according to the present disclosure may exhibit other effects apparent to those skilled in the art from the description of the present specification in addition to or instead of the above effects.

### Reference Signs List

1 MEDICAL OBSERVATION SYSTEM
10 ROBOT ARM DEVICE
11 ARM PORTION
11a JOINT PORTION
12 IMAGING UNIT
13 LIGHT SOURCE UNIT
20 CONTROL UNIT
21 IMAGE PROCESSING UNIT
22 IMAGING CONTROL UNIT
23 ARM CONTROL UNIT
25 RECEIVING UNIT
26 DISPLAY CONTROL UNIT
40 PRESENTING DEVICE
60, 130 STORAGE UNIT
100 LEARNING MODEL GENERATION UNIT
110 EXTERNAL INPUT INFORMATION ACQUISITION UNIT
120 AUTONOMOUS OPERATION LEARNING MODEL GENERATION UNIT
122 AUTONOMOUS OPERATION RULE GENERATION UNIT
124 IDEAL MAP GENERATION UNIT
132 LEARNING MODEL
134 RULE-BASED CONTROL MODEL
135 IDEAL BODY INTERNAL ENVIRONMENT MAP
136a, 136b, 136c, 138, 600, 602, 606, 608, 610, 612 IMAGE
140 OUTPUT UNIT
200 AUTONOMOUS OPERATION EXECUTION UNIT
210 CONTROL UNIT
212 INPUT INFORMATION ACQUISITION UNIT
214 AUTONOMOUS OPERATION GENERATION UNIT
216, 272 AUTONOMOUS OPERATION CORRECTION UNIT
218 CAMERA PREDICTION VISUAL FIELD CALCULATION UNIT
220 VISUAL FIELD LINKED CONTROL CALCULATION UNIT
224 USER INSTRUCTION RECONCILIATION UNIT
226 ARM CONTROL UNIT
228 TIP CONTROL UNIT
230 CUT-OUT VISUAL FIELD GENERATION UNIT
250 SIMULATOR UNIT
252 INPUT INFORMATION RECEIVING UNIT
254 IDEAL ENVIRONMENT MAP HOLDING UNIT
256 BODY INTERNAL ENVIRONMENT MAP GENERATION UNIT
258 IDEAL ENVIRONMENT INPUT CORRECTION UNIT
260 IDEAL ENVIRONMENT AUTONOMOUS OPERATION GENERATION UNIT
262 IDEAL ENVIRONMENT SIMULATION UNIT
264 CURRENT ENVIRONMENT AUTONOMOUS OPERATION GENERATION UNIT
266 CURRENT ENVIRONMENT SIMULATION UNIT
268 AUTONOMOUS OPERATION RESULT COMPARISON UNIT
270 AUTONOMOUS OPERATION CORRECTION PARAMETER GENERATION UNIT
274 CURRENT ENVIRONMENT SIMULATION UPDATE UNIT
276 INTERFERENCE PREDICTION UNIT
278 IMAGE GENERATION UNIT
402 ACTUAL IMAGE ACQUISITION UNIT
404 VIRTUAL IMAGE ACQUISITION UNIT
406 PREDICTION RESULT GENERATION UNIT
500 DIFFERENCE
502 CORRECTION AMOUNT
504 DIFFERENCE
506 PARAMETER
630 VISUAL FIELD RANGE

## Claims

1. A medical arm control system (20) comprising:
circuitry (23) configured to
generate autonomous operation control information to autonomously operate a medical arm (10) based on external input information; simulate an operation performed using the medical arm;
wherein the circuitry is configured to
simulate the operation by a first simulation of the operation of the medical arm in an actual environment obtained from the external input information and a second simulation of the operation of the medical arm in a reference environment, and
correct the autonomous operation control information in real time based on the first simulation and the second simulation.

2. The medical arm control system according to claim 1, wherein the circuitry is further configured to
extract a difference between the first simulation and the second simulation, and
correct the autonomous operation control information in real time based on the difference.

3. The medical arm control system according to any one of the preceding claims, wherein the external input information includes position information and posture information of the medical arm in an actual environment.

4. The medical arm control system according to any one of the preceding claims, wherein the medical arm further comprises a support for a medical camera.

5. The medical arm control system according to claim 4, wherein the external input information includes an image of the actual environment taken by the medical camera.

6. The medical arm control system according to claim 4, wherein the medical camera is an endoscope.

7. The medical arm control system according to claim 3, wherein the external input information includes position information and posture information of a medical instrument in the actual environment.

8. The medical arm control system according to claim 7, wherein the medical arm further comprises a support for the medical instrument.

9. The medical arm control system according to any one of the preceding claims, wherein the external input information includes sensing data obtained by a distance sensor.

10. The medical arm control system according to claim 9, wherein the distance sensor is a stereo sensor, a time-of-flight sensor, or a structured light sensor.

11. The medical arm control system according to claim 1, wherein the autonomous operation control information includes target position information and target posture information of the medical arm.

12. The medical arm control system according to any one of the preceding claims, wherein the circuitry is configured to generate the autonomous operation control information based on a learning model obtained by machine learning.

13. The medical arm control system according to any one of the preceding claims, wherein the circuitry is further configured to control the medical arm based on the corrected autonomous operation control information.

14. The medical arm control system according to any one of the preceding claims, further comprising the medical arm.

15. The medical arm control system according to claim 14, wherein medical arm includes a plurality of medical arms to be controlled by the circuitry.

## Patentansprüche

1. Steuerungssystem (20) für medizinische Arme, umfassend:
Schaltung (23), die konfiguriert ist zum
Erzeugen von Informationen zur autonomen Betriebssteuerung, um einen medizinischen Arm (10) basierend auf externen Eingabeinformationen autonom zu betreiben;
Simulieren einer unter Verwendung des medizinischen Arms durchgeführten Operation;
wobei die Schaltung konfiguriert ist, um
die Operation durch eine erste Simulation des Betriebs des medizinischen Arms in einer tatsächlichen Umgebung, die aus den externen Eingangsinformationen erhalten wird, und eine zweite Simulation des Betriebs des medizinischen Arms in einer Referenzumgebung zu simulieren, und
die autonome Betriebssteuerungsinformation in Echtzeit basierend auf der ersten Simulation und der zweiten Simulation zu korrigieren.

2. Steuerungssystem für medizinische Arme nach Anspruch 1, wobei die Schaltung ferner konfiguriert ist zum
Extrahieren eines Unterschieds zwischen der ersten Simulation und der zweiten Simulation, und
Korrigieren der autonomen Betriebssteuerungsinformationen in Echtzeit basierend auf der Differenz.

3. Steuerungssystem für medizinische Arme nach einem der vorstehenden Ansprüche, wobei die externen Eingabeinformationen Positionsinformationen und Haltungsinformationen des medizinischen Arms in einer tatsächlichen Umgebung einschließen.

4. Steuerungssystem für medizinische Arme nach einem der vorstehenden Ansprüche, wobei der medizinische Arm ferner eine Halterung für eine medizinische Kamera umfasst.

5. Steuerungssystem für medizinische Arme nach Anspruch 4, wobei die externen Eingabeinformationen ein Bild der tatsächlichen Umgebung einschließen, das von der medizinischen Kamera aufgenommen wurde.

6. Steuerungssystem für medizinische Arme nach Anspruch 4, wobei die medizinische Kamera ein Endoskop ist.

7. Steuerungssystem für medizinische Arme nach Anspruch 3, wobei die externen Eingabeinformationen Positionsinformationen und Haltungsinformationen eines medizinischen Instruments in der tatsächlichen Umgebung einschließen.

8. Steuerungssystem für medizinische Arme nach Anspruch 7, wobei der medizinische Arm ferner eine Halterung für das medizinische Instrument umfasst.

9. Steuerungssystem für medizinische Arme nach einem der vorstehenden Ansprüche, wobei die externen Eingabeformationen Erfassungsdaten einschließen, die von einem Abstandssensor erhalten werden.

10. Steuerungssystem für medizinische Arme nach Anspruch 9, wobei der Abstandssensor ein Stereosensor, ein Laufzeitsensor oder ein Sensor für strukturiertes Licht ist.

11. Steuerungssystem für medizinische Arme nach Anspruch 1, wobei die Informationen zur autonomen Betriebssteuerung Zielpositionsinformationen und Zielhaltungsinformationen des medizinischen Arms einschließen.

12. Steuerungssystem für medizinische Arme nach einem der vorstehenden Ansprüche, wobei die Schaltung konfiguriert ist, um die Informationen zur autonomen Betriebssteuerung basierend auf einem durch maschinelles Lernen erhaltenen Lernmodell zu erzeugen.

13. Steuerungssystem für medizinische Arme nach einem der vorstehenden Ansprüche, wobei die Schaltung ferner konfiguriert ist, um den medizinischen Arm basierend auf den korrigierten Informationen zur autonomen Betriebssteuerung zu steuern.

14. Steuerungssystem für medizinische Arme nach einem der vorstehenden Ansprüche, ferner umfassend den medizinischen Arm.

15. Steuerungssystem für medizinische Arme nach Anspruch 14, wobei der medizinische Arm eine Vielzahl von medizinischen Armen einschließt, die durch die Schaltung gesteuert werden sollen.

## Revendications

1. Système de commande de bras médical (20), comprenant :
un ensemble de circuits (23) configurés pour
générer des informations de commande de fonctionnement autonome pour faire fonctionner de manière autonome un bras médical (10) sur la base d'informations d'entrée externes ;
simuler une opération effectuée à l'aide du bras médical ;
dans lequel l'ensemble de circuits sont configurés pour
simuler le fonctionnement par une première simulation du fonctionnement du bras médical dans un environnement réel obtenu à partir des informations d'entrée externes et une seconde simulation du fonctionnement du bras médical dans un environnement de référence, et
corriger les informations de commande de fonctionnement autonome en temps réel sur la base de la première simulation et de la seconde simulation.

2. Système de commande de bras médical selon la revendication 1, dans lequel l'ensemble de circuits sont configurés en outre pour
extraire une différence entre la première simulation et la seconde simulation, et
corriger les informations de commande de fonctionnement autonome en temps réel sur la base de la différence.

3. Système de commande de bras médical selon l'une quelconque des revendications précédentes, dans lequel les informations d'entrée externes comportent des informations de position et des informations de posture du bras médical dans un environnement réel.

4. Système de commande de bras médical selon l'une quelconque des revendications précédentes, dans lequel le bras médical comprend en outre un support pour une caméra médicale.

5. Système de commande de bras médical selon la revendication 4, dans lequel les informations d'entrée externes comportent une image de l'environnement réel prise par la caméra médicale.

6. Système de commande de bras médical selon la revendication 4, dans lequel la caméra médicale est un endoscope.

7. Système de commande de bras médical selon la revendication 3, dans lequel les informations d'entrée externes comportent des informations de position et des informations de posture d'un instrument médical dans l'environnement réel.

8. Système de commande de bras médical selon la revendication 7, dans lequel le bras médical comprend en outre un support pour l'instrument médical.

9. Système de commande de bras médical selon l'une quelconque des revendications précédentes, dans lequel les informations d'entrée externes comportent des données de détection obtenues par un capteur de distance.

10. Système de commande de bras médical selon la revendication 9, dans lequel le capteur de distance est un capteur stéréo, un capteur de temps de vol ou un capteur de lumière structurée.

11. Système de commande de bras médical selon la revendication 1, dans lequel les informations de commande de fonctionnement autonome comportent des informations de position cible et des informations de posture cible du bras médical.

12. Système de commande de bras médical selon l'une quelconque des revendications précédentes, dans lequel l'ensemble de circuits sont configurés pour générer les informations de commande de fonctionnement autonome sur la base d'un modèle d'apprentissage obtenu par apprentissage automatique.

13. Système de commande de bras médical selon l'une quelconque des revendications précédentes, dans lequel l'ensemble de circuits sont en outre configurés pour commander le bras médical sur la base des informations de commande de fonctionnement autonome corrigées.

14. Système de commande de bras médical selon l'une quelconque des revendications précédentes, comprenant en outre le bras médical.

15. Système de commande de bras médical selon la revendication 14, dans lequel le bras médical comporte une pluralité de bras médicaux à commander par l'ensemble de circuits.
